# EUROPEAN PATENT SPECIFICATION

(11) **EP 1 353 953 B1**
(45) Date of publication and mention of the grant of the patent: **29.11.2006**
(21) Application number: 01937340.6
(22) Date of filing: 11.05.2001
(51) Int. Cl.: C07K 14/715, C12N 15/12, C12N 15/66, A61K 38/17, C07K 16/28

(54) **MU-1, MEMBER OF THE CYTOKINE RECEPTOR FAMILY**
MU-1, EIN MITGLIED DER CYTOKINE REZEPTOR FAMILIE
MU-1, MEMBRE DE LA FAMILLE DES RECEPTEURS DE LA CYTOKINE

(30) Priority: 11.05.2000 US 569384
(43) Date of publication of application: 22.10.2003
(73) Proprietor: Genetics Institute, LLC, Cambridge, MA 02140 (US)
(72) Inventor: DONALDSON, Debra, D., Medford, MA 02155 (US); UNGER, Michelle, J., Brighton, MA 02135 (US); YOUNG, Deborah, A., Melrose, MA 02176 (US); WHITTERS, Matthew, J., Hudson, MA 01749 (US); LOWE, Leslie, Sudbury, MA 01776 (US); COLLINS, Mary, Natick, MA 01760 (US)
(74) Representative: Dörries, Hans Ulrich
(86) International application number: PCT/US2001/015395
(87) International publication number: WO 2001/085792

(56) References cited:
- EP-A- 1 088 831
- WO-A-00/17235
- WO-A-00/69880
- WO-A-01/77171
- WO-A-99/47675
- LAI CHUN-FAI ET AL: "STAT3 and STAT5B are targets of two different signal pathways activated by hematopoietin receptors and control transcription via separate cytokine response elements." JOURNAL OF BIOLOGICAL CHEMISTRY, vol. 270, no. 40, 1995, pages 23254-23257, XP001064408 ISSN: 0021-9258
- DATABASE EMBL [Online] 18 July 2000 (2000-07-18) Database accession no. AF279436 XP002194003
- DATABASE EMBL [Online] 23 September 2000 (2000-09-23) Database accession no. AB049137 XP002194004
- PARRISH-NOVAK JULIA ET AL: "Interleukin 21 and its receptor are involved in NK cell expansion and regulation of lymphocyte function." NATURE (LONDON), vol. 408, no. 6808, October 2000 (2000-10), pages 57-63, XP002194001 ISSN: 0028-0836
- OZAKI KATSUTOSHI ET AL: "Cloning of a type I cytokine receptor most related to the IL-2 receptor beta chain." PROCEEDINGS OF THE NATIONAL ACADEMY OF SCIENCES OF THE UNITED STATES, vol. 97, no. 21, 10 October 2000 (2000-10-10), pages 11439-11444, XP002194002 October 10, 2000 ISSN: 0027-8424

## Description

### Field of the Invention

The present invention relates to new members of the mammalian cytokine receptor family of proteins (including without limitation human and murine receptor proteins), fragments thereof, recombinant polynucleotides and cells useful for expressing such proteins, and methods for identifying compounds capable of treating immune disorders comprising assaying the ability of the compounds to modulate interaction of the proteins with a STAT molecule.

### Background of the Invention

A variety of regulatory molecules, known as hematopoietins, have been identified which are involved in the development and proliferation of the various populations of hematopoietic or blood cells. Most hematopoietins exhibit certain biological activities by interacting with a receptor on the surface of target cells. Cytokine receptors are commonly composed of one, two or three chains. Many cytokine receptors and some cytokines, such as IL-12 p40, are members of the hematopoietin receptor superfamily of proteins. Identification of new members of the hematopoietin receptor superfamily can be useful in regulation of hematopoiesis, in regulation of immune responses and in identification of other members of the hematopoietin superfamily, including cytokines and receptors.

It would be desirable to identify and determine the DNA and protein sequence for heretofore unknown members of the hematopoietin receptor superfamily.

### Summary of the Invention

In accordance with the present invention, a method for identifying a compound capable of treating an immune disorder comprising assaying the ability of the compound to modulate MU-1 polypeptide interaction with a STAT molecule, thereby identifying a compound capable of treating an immune disorder, is disclosed. The STAT signaling pathway can, for example be a STAT 3 signaling pathway or a STAT 5 signaling pathway. The invention also provides a method of identifying a modulator of MU-1 activity comprising contacting a cell expressing a MU-1 protein according to SEQ ID NO:2 or 10, or a portion thereof capable of interaction with STAT molecules, with a test compound and determining the ability of said test compound to modulate interaction of said MU-1 protein, or said portion thereof, with a STAT molecule. Said STAT molecule can, for example be STAT 3 or STAT 5. Further, in this method the MU-1 protein can, for example, be according to SEQ ID NO:9, and can be expressed in said cell as a fusion of the MU-1 intracellular domain and the human BPO receptor extracellular domain.

Also disclosed are polynucleotides encoding the MU-1 hematopoietin receptor superfamily chain, including without limitation those from the murine and human sources.

In certain embodiments, an isolated polynucleotide comprises a nucleotide sequence selected from the group consisting of:
(a) the nucleotide sequence of SEQ ID NO:1;
(b) the nucleotide sequence of SEQ ID NO:1 from nucleotide 238 to nucleotide 1852;
(c) the nucleotide sequence of SEQ ID NO:1 from nucleotide 301 to nucleotide 1852;
(d) the nucleotide sequence of SEQ ID NO:1 from nucleotide 301 to nucleotide 945;
(e) a nucleotide sequence varying from the sequence of the nucleotide sequence specified in any of (a)-(d) as a result of degeneracy of the genetic code;
(f) a nucleotide sequence capable of hybridizing under stringent conditions to the nucleotide specified in any of (a)-(d);
(g) a nucleotide sequence encoding a species homologue of the sequence of SEQ ID NO:2; and
(h) an allelic variant of the nucleotide sequence specified in any of (a)-(d). Preferably, the nucleotide sequence encodes a protein having a biological activity of the MU-1 hematopoietin receptor superfamily chain. The nucleotide sequence may be operably linked to an expression control sequence.

Also disclosed are isolated polynucleotides comprising a nucleotide sequence encoding a peptide or protein comprising an amino acid sequence selected from the group consisting of:
(a) the amino acid sequence of SEQ ID NO:2;
(b) the amino acid sequence of SEQ ID NO:2 from amino acids 22 to 538;
(c) the amino acid sequence of SEQ ID NO:2 from amino acids 22 to 236;
(d) the amino acid sequence of SEQ ID NO:2 from amino acids 1 to 236; and
(e) fragments of (a)-(d) having a biological activity of the MU-1 hematopoietin receptor superfamily chain.

Further provided is an isolated polynucleotide comprising a nucleotide sequence selected from the group consisting of:
(a) the nucleotide sequence of SEQ ID NO:9;
(b) a nucleotide sequence varying from the sequence of the nucleotide sequence specified in (a) as a result of degeneracy of the genetic code;
(c) a nucleotide sequence capable of hybridizing under stringent conditions to the nucleotide specified in (a);
(d) an allelic variant of the nucleotide sequence specified in (a).

Further disclosed are isolated polynucleotides comprising a nucleotide sequence encoding a peptide or protein comprising an amino acid sequence selected from the group consisting of:
(a) the amino acid sequence of SEQ ID NO: 10;
(b) fragments of (a) having a biological activity of the MU-1 hematopoietin receptor superfamily chain.

Host cells, preferably mammalian cells, transformed with the polynucleotides are also provided.

In other embodiments, the invention provides a process for producing a MU-1 protein. The process comprises:
(a) growing a culture of the host cell of the present invention in a suitable culture medium; and
(b) purifying the human MU-1 protein from the culture.
Proteins produced according to these methods are also provided.

The present invention also provides for an isolated MU-1 protein comprising an amino acid sequence selected from the group consisting of:
(a) the amino acid sequence of SEQ ID NO:2;
(b) the amino acid sequence of SEQ ID NO:2 from amino acids 22 to 538;
(c) the amino acid sequence of SEQ ID NO:2 from amino acids 22 to 236;
(d) the amino acid sequence of SEQ ID NO:2 from amino acids 1 to 236; and
(e) fragments of (a)-(d) having a biological activity of the MU-1 hematopoietin receptor superfamily chain.

Also disclosed is an isolated MU-1 protein comprising an amino acid sequence selected from the group consisting of:
(a) the amino acid sequence of SEQ ID NO: 10;
(b) fragments of (a) having a biological activity of the MU-1 hematopoietin receptor superfamily chain.

The amino acid sequence of Murine MU-1 (SEQ ID NO:10) is about 65% identical to the amino acid sequence of human MU-1.

In other preferred embodiments, the specified amino acid sequence is part of a fusion protein (with an additional amino acid sequence not derived from MU-1). Preferred fusion proteins comprise an antibody fragment, such as an Fc fragment.

Pharmaceutical compositions comprising an MU-1 protein and a pharmaceutically acceptable carrier are also provided.

Also disclosed are compositions comprising an antibody which specifically reacts with a protein of the present invention.

A MU-1 nucleic acid molecule as described herein is at least 65%, 70%, 75%, 80%, 85%, 86%, 87%, 88%, 89%, 90%, 91%, 92%, 93%, 94%, 95%, 96%, 97%, 98%, 99%, 99.5% or more identical to the nucleotide sequence (*e.g.,* to the entire length of the nucleotide sequence) shown in SEQ ID NO:1 or 9.

Preferably, the nucleic acid molecule consists of the nucleotide sequence shown in SEQ ID NO:1 or 9. It is also preferred that the nucleic acid molecule includes a fragment of at least 50,100, 200, 300, 400, 500, 600, 700, 800, 900, 1000, 1100, 1200, 1300, 1400, 1500, 1600, 1700, 1800, or more nucleotides (e.g., contiguous nucleotides) of the nucleotide sequence of SEQ ID NO:1 or 9, or a complement thereof.

Preferably, the MU-1 protein family member has an amino acid sequence at least about 65%, 70%, 75%, 80%, 85%, 86%, 87%, 88%, 89%, 90%, 91%, 92%, 93%, 94%, 95%, 96%, 97%, 98%, 99%, 99.5% or more identical to the amino acid sequence of SEQ ID NO:2 or 10.

Also disclosed are fragments of the protein having the amino acid sequence of SEQ ID NO:2 or 10, wherein the fragment comprises at least 15, 20, 30, 40, 50, 60, 70, 80, 90, or 100 amino acids (*e.g*., contiguous amino acids) of the amino acid sequence of SEQ ID NO:2 or 10.

In another aspect, a method for detecting the presence of a MU-1 nucleic acid molecule, protein or polypeptide in a biological sample by contacting the biological sample with an agent capable of detecting a MU-1 nucleic acid molecule, protein or polypeptide such that the presence of a MU-1 nucleic acid molecule, protein or polypeptide is detected in the biological sample is provided.

In another aspect, a method for detecting the presence of MU-1 activity in a biological sample by contacting the biological sample with an agent capable of detecting an indicator of MU-1 activity such that the presence of MU-1 activity is detected in the biological sample is provided.

In another aspect, a method for modulating MU-1 activity comprising contacting a cell capable of expressing MU-1 with an agent that modulates MU-1 activity such that MU-1 activity in the cell is modulated is disclosed. In one embodiment, the agent inhibits MU-1 activity. In another embodiment, the agent stimulates MU-1 activity. In one embodiment, the agent is an antibody that specifically binds to a MU-1 protein. In another embodiment, the agent modulates expression of MU-1 by modulating transcription of a MU-1 gene or translation of a MU-1 mRNA. In yet another embodiment, the agent is a nucleic acid molecule having a nucleotide sequence that is antisense to the coding strand of a MU-1 mRNA or a MU-1 gene.

The methods disclosed can be used to treat a subject having a disorder characterized by aberrant or unwanted MU-1 protein or nucleic acid expression or activity (e.g., an MU-1 associated disorder) by administering an agent which is a MU-1 modulator to the subject. In one embodiment, the MU-1 modulator is an MU-1 protein. In another embodiment the MU-1 modulator is an MU-1 nucleic acid molecule. In yet another embodiment, the MU-1 modulator is a peptide, peptidomimetic, antibody, or other small molecule.

Also provided are diagnostic assays for identifying the presence or absence of a genetic alteration characterized by at least one of (i) aberrant modification or mutation of a gene encoding a MU-1 protein; (ii) mis-regulation of the MU-1 gene; and (iii) aberrant post-translational modification of an MU-1 protein, wherein a wild-type form of the gene encodes a protein with a MU-1 activity.

In another aspect methods for identifying a compound that binds to or modulates the activity of a MU -1 protein are provided, by providing an indicator composition comprising a MU-1 protein having MU-1 activity, contacting the indicator composition with a test compound, and determining the effect of the test compound on MU-1 activity (e.g., modulation of STAT phosphorylation, e.g., STAT 3 or STAT 5 phosporylation) in the indicator composition to identify a compound that modulates the activity of a MU-1 protein.

### Brief Description of the Drawings

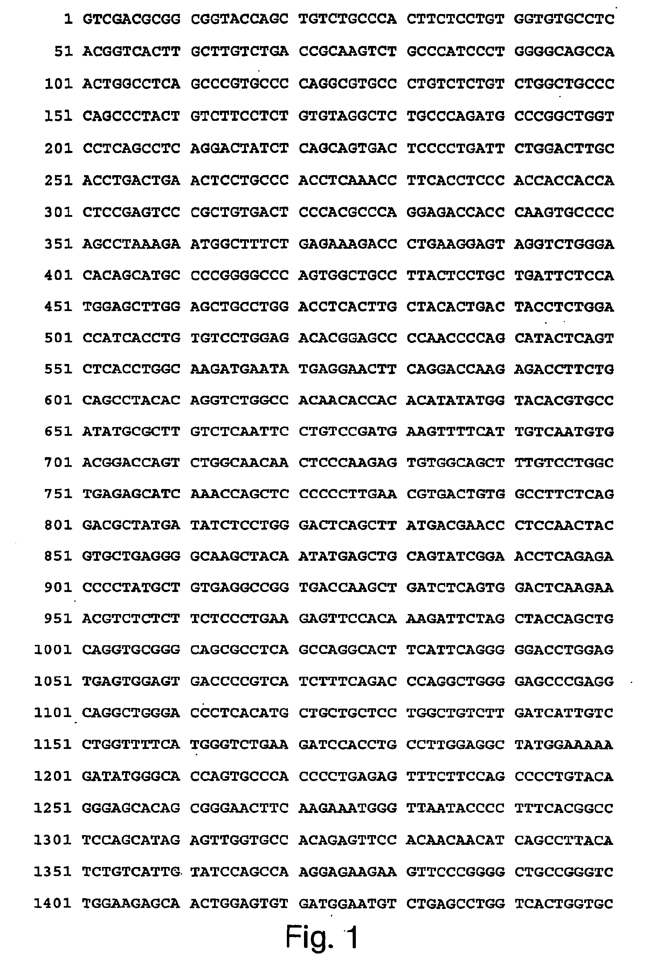

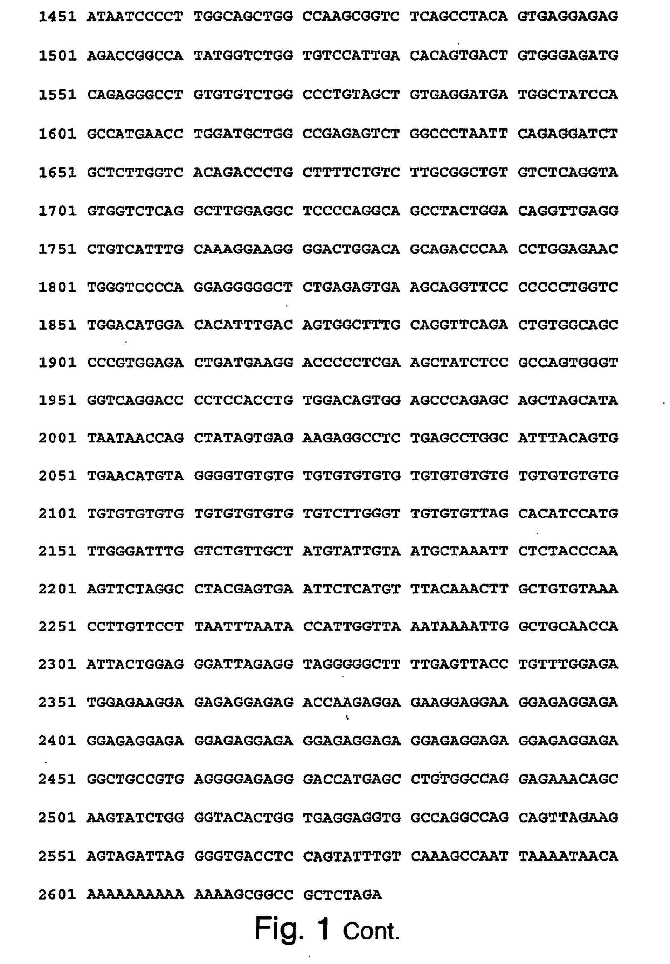

*Figure 1* depicts the full-length cDNA sequence of murine MU-1. The nucleotide sequence corresponds to nucleotides 1-2628 of SEQ ID NO:9.

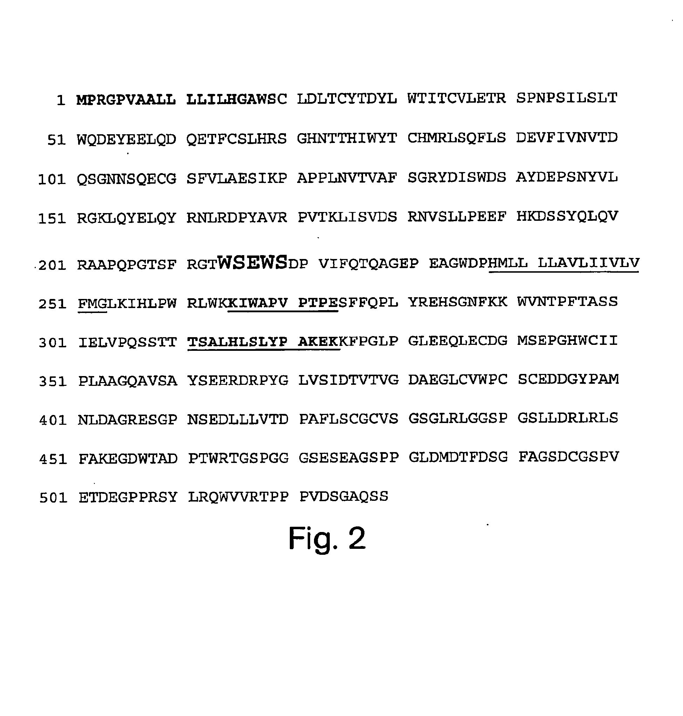

*Figure 2* depicts the amino acid sequence of murine MU-1 (corresponding to the amino acids 1-529 of SEQ ID NO:10). There is a predicted leader sequence at amino acids 1-19, which was predicted by SPScan with score of 10.1 (bold-face type). There is a predicted transmembrane domain at amino acids 237-253 (underlined). Predicted signaling motifs include the following regions: Box 1: amino acids 265-274 and Box 2: amino acids 310-324 (bold and underlined); Six Tyrosines are located at amino acid positions 281, 319, 361, 368, 397, and 510. The WSXWS motif (SEQ ID NO:8) is located at aminio acid residue 214 to amino acid residue 218 (in large, bold-face type). Potential STAT docking sites include amino acids 393-398 and amino acids 510-513.

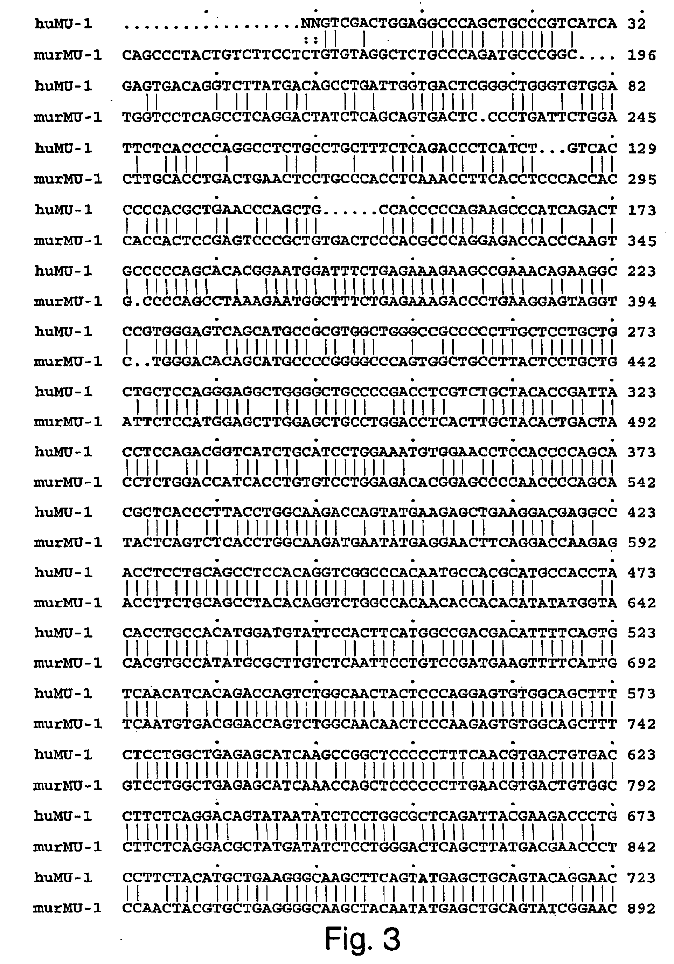

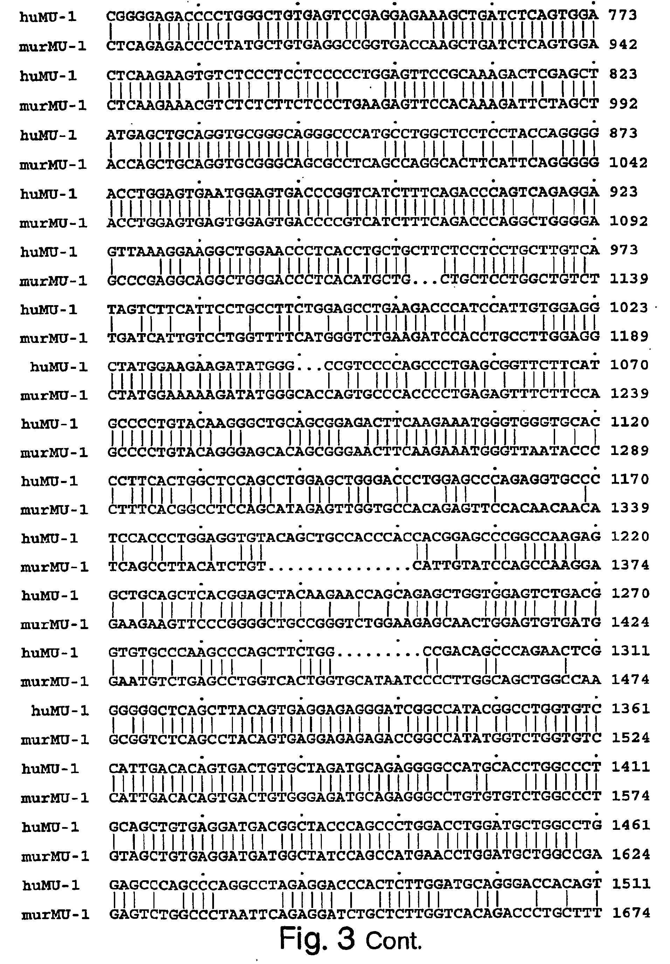

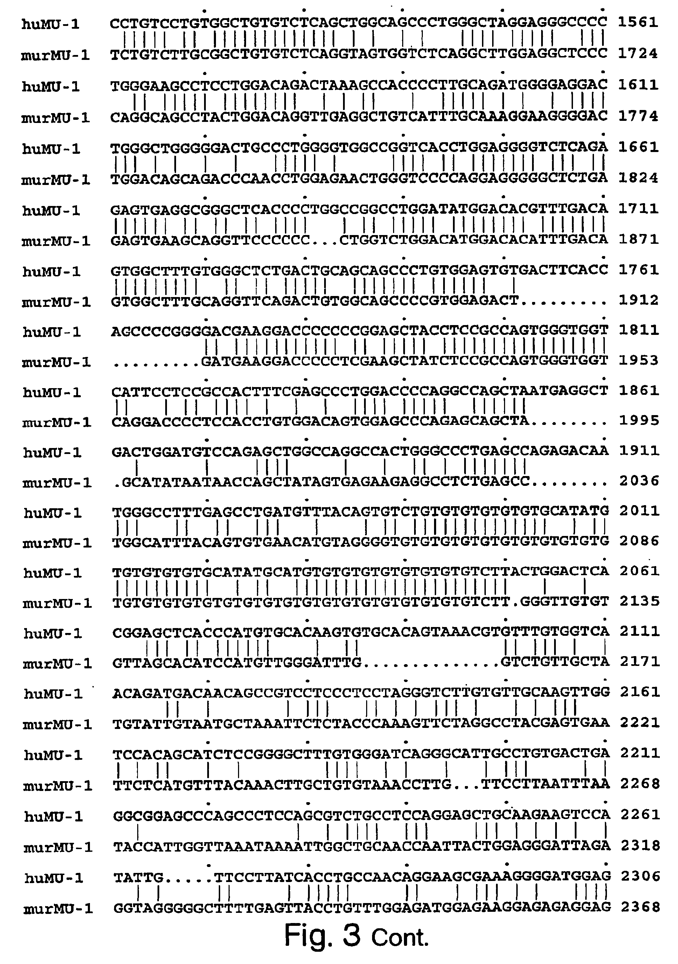

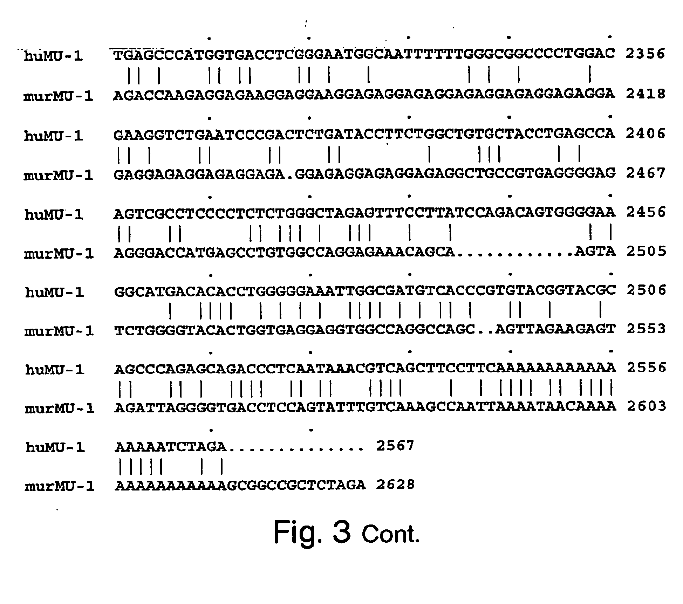

*Figure 3* depicts the GAP comparison of human and murine MU-1 cDNA sequences (corresponding to nucleic acids 1-2665 of SEQ ID NO:1 and nucleic acids 1-2628 of SEQ ID NO:9, respectively). HuMU-1= human MU-1, murMU-1= murine MU-1. Gap Parameters: Gap Weight= 50, Average Match= 10.000, Length Weight= 3, Average Mismatch= 0.000. Percent Identity= 66.116.

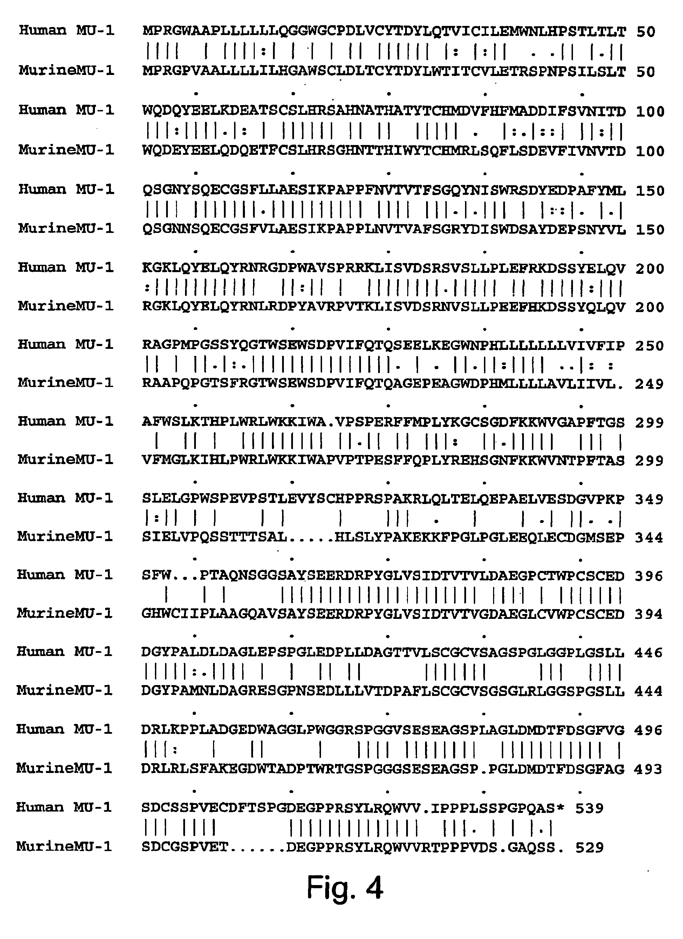

*Figure 4* depicts a GAP comparison of the human MU-1 protein (corresponding to amino acids 1-538 of SEQ ID NO:2) and the murine MU-1 protein (corresponding to amino acids 1-529 of SEQ ID NO:10). BLOSUM62 amino acid substitution matrix. (Henikoff, S. and Henikoff, J. G. (1992)). Amino acid substitution matrices from protein blocks (Proc. Natl. Acad. Sci. USA 89: 10915-10919). Gap parameters= Gap Weight: 8, Average Match= 2.912, Length Weight= 2, Average Mismatch= -2.003. Percent Identity= 65.267

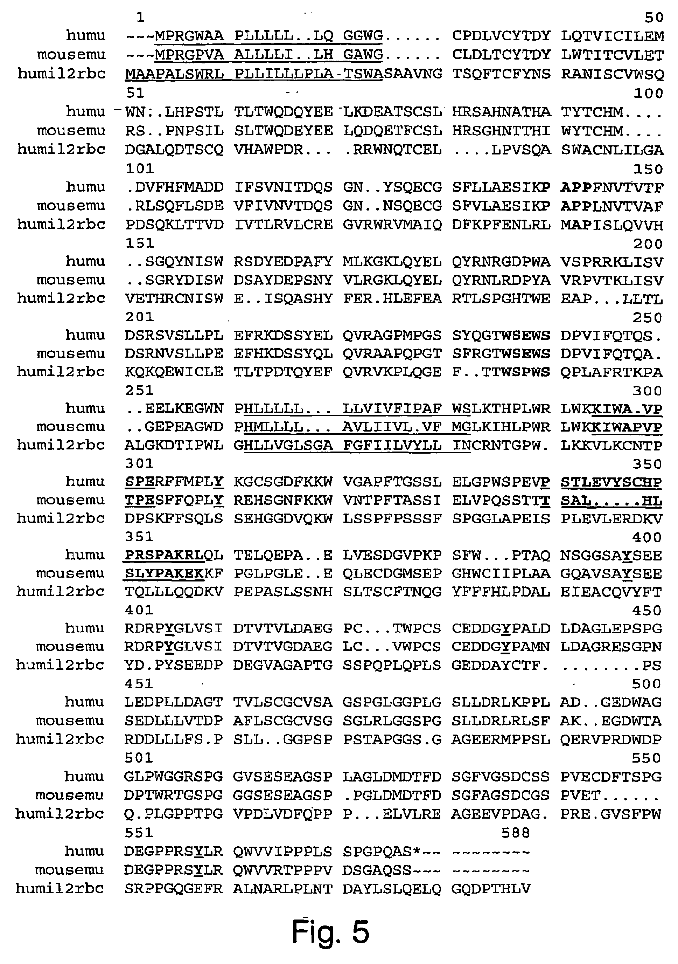

*Figure 5* depicts a multiple sequence alignment of the amino acids of human MU-1 (corresponding to SEQ ID NO:2), murine MU-1 (corresponding to SEQ ID NO:10), and human IL2beta chain (GENbank Accession No. M26062). Leader and transmembrane domains are underlined. Conserved cytokine receptor module motifs are indicated by bold-face type. Potential signaling regions are indicated by underlining and bold-face type.

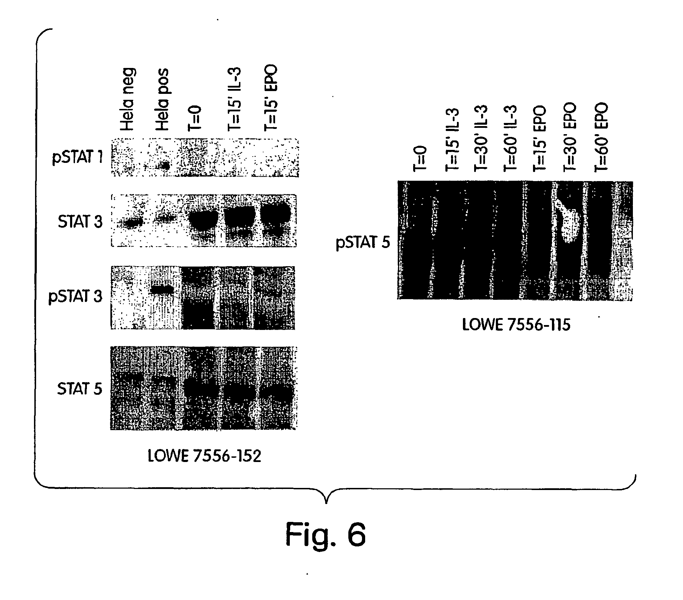

*Figure 6* depicts signaling through MU-1. MU-1 phosphorylates STAT 5 in Clone E7 EPO-MU-1 chimera. Under the conditions specified in Example 3, signaling through MU-1 results in the phosphorylation of STAT 5 at all time-points tested. Treatment of controls or the chimeric BAF-3 cells with IL-3 resulted in phosphorylation of STAT 3, but not STAT 1 or 5.

### Detailed Description of Preferred Embodiment

The inventors of the present application have provided polynucleotides encoding the MU-1 hematopoietin receptor superfamily chain (hereinafter "MU-1" or "MU-1 protein"), including without limitation polynucleotides encoding human and murine MU-1.

In a particularly preferred embodiment, the MU-1 protein and nucleic acid molecules are human MU-1 molecules. A 70 amino acid region of the human IL5 receptor (LMTNAFISIIDDLSKYDVQVRAAVSSMCREAGLWSEWSQPIYVGND EHKPLREWFVIVIMATICFILLIL, SEQ ID NO:3) was used to search the GenBank EST database using the TBLASTN algorithm. Sequence within the genomic BAC clone AC002303 from human chromosome 16p12 was identified with homology to this region, suggesting that this segment might encode a gene for a novel hematopoietin receptor. Examination of open reading frames within 1000bp of nucleotide 40,886 revealed a 270bp open frame which when used in a BLASTP search of GenPept exclusively identified members of the cytokine receptor family. A stop codon present at the end of this reading frame was interpreted as indication of transition over an exon/intron border.

It was then determined whether RNA was transcribed from a gene contained within this BAC clone from chromosome 16p12. PCRprimers were synthesized based on the largest ORF segment which contained peptide sequence conserved within the cytokine receptor family. Primers GAGTCCGAGGAGAAAGCTGATCTCA (5p) (SEQ ID NO:4) and GAAAGATGACCGGGTCACTCCATT (3p) (SEQ ID NO:5) were used in PCRs to screen phage libraries from various human tissues (Clontech). PCR products of the expected 164 bp size which specifically hybridized to a 32-P labeled oligonucleotide of the sequence ACTCGAGCTATGAGCTGCAGGTGCGGGCA (SEQ ID NO:6) were observed in phage from lung, kidney, placenta and heart. Using the oligonucleotide ACTCGAGCTATGAGCTGCAGGTGCGGGCA (SEQ ID NO:7) a full-length cDNA clone NN14-1b (MU-1) was identified, purified, and sequenced. The DNA sequence and the predicted amino acid sequence are shown in SEQ ID NO:1 and SEQ ID NO:2, respectively. The predicted amino acid sequence of the human MU-1 receptor chain includes a putative signal sequence from amino acids 1-21. The mature human MU-1 is believed to have the sequence of amino acids 24-538 of SEQ ID NO:2. A transmembrane domain is found at amino acids 237-254.

In another embodiment, the MU-1 protein and nucleic acid molecules are murine MU-1 molecules. To identify the polynucleotide sequence encoding the murine MU-1 protein a partial fragment of the murine homolog of the MU-1 receptor was isolated by PCR from mouse cDNA using oligonucleotides derived from the human sequence, as described in Example 1. The DNA sequence of this fragment was determined, and two oligonucleotides were derived from an internal portion of this fragment with the following sequences:
TTGAACGTGACTGTGGCCTT (5p) (SEQ ID NO: 13)
TGAATGAAGTGCCTGGCTGA (3p) (SEQ ID NO: 14).
The oligonucleotides were used to amplify an internal 262 nucleotide fragment of the original PCR product (corresponding to nucleotides 781-1043 of the murine cDNA sequence of figure 1 and SEQ ID NO:9) to use as a hybridization probe to screen a cDNA library isolated from the 2D6 T cell line. DNA sequence was determined from two independent clones. Clone 6 was sequenced and confirmed to be the full-length murine homolog of human MU-1.

The full-length nucleotide sequence of murine MU-1 is shown in figure 1 (corresponding to nucleotides 1-2628 of SEQ ID NO:9). The nucleotide sequence has a predicted leader sequence at nucleotides 407-464, coding sequence at nucleotides 407-1993, and a termination codon at nucleotides 1994-1997. Nucleotides 1-406 correspond to the 5' untranslated region and nucleotides 1998-2628 correspond to the 3' untranslated region. The predicted protein sequence of murine MU-1 is shown in figure 2 (corresponding to amino acids 1-529 of SEQ ID NO:10).

The murine MU-1 protein contains a predicted leader sequence determined by SPScan (score=10.1) (corresponding to amino acids 1-19 of SEQ ID NO:10), and a predicted transmembrane domain (corresponding to amino acids 237-253 of SEQ ID NO:10). Predicted signaling motifs include the following regions: Box 1: amino acids 265-274 of SEQ ID NO:10, Box 2: amino acids 310-324 of SEQ ID NO:10, six tyrosine residues at positions 281, 319, 361, 368, 397, and 510 of SEQ ID NO:10. Potential STAT docking sites include but are not limited to: STAT 5: EDDGYPA, STAT 3:YLQR.

The open reading frame of MU-1 encodes a member of the hematopoietin receptor family. In one embodiment, MU-1 has a leader sequence, conserved cysteine pairs, PP, and WSXWS (SEQ ID NO:8) motifs characteristic of the family, as well as a transmembrane domain and an extensive cytoplasmic domain. MU-1 also contains a conserved PXPP as well as Box I and Box II signaling motifs in the cytoplasmic domain (see Figure 5). These domains are conserved between murine MU-1 and human MU-1. Subsequent FASTA alignment of MU-1 sequence with GenPept showed greatest homology with human IL-2Rb (see figure 5).

The human MU-1 cDNA was deposited with the American Type Culture Collection on March 10,1998, as accession number ATCC 98687.

By Northern analysis, as described in Example 4, murine MU-1 was detected in adult murine spleen, lung, and heart tissues. Human MU-1 was detected in adult human lymphoid tissues, PBLs, thymus, spleen and lymph node, and in fetal lung.

Any forms of MU-1 proteins of less than full length are encompassed within the present invention and are referred to herein collectively with full length and mature forms as "MU-1" or "MU-1 proteins." MU-1 proteins of less than full length may be produced by expressing a corresponding fragment of the polynucleotide encoding the full-length MU-1 protein (SEQ ID NO:4 or SEQ ID NO:6). These corresponding polynucleotide fragments can also be used. Modified polynucleotides as described above may be made by standard molecular biology techniques, including construction of appropriate desired deletion mutants, site-directed mutagenesis methods or by the polymerase chain reaction using appropriate oligonucleotide primers.

For the purposes of the present invention, a protein has "a biological activity of the MU-1 hematopoietin receptor superfamily chain" if it possesses one or more of the biological activities of the corresponding mature MU-1 protein. MU-1 activity includes interaction with STAT molecules (e.g., STAT 5, STAT 3).

MU-1 or active fragments thereof (MU-1 proteins) may be fused to carrier molecules such as immunoglobulins or immunoglobulin fragment. For example, soluble forms of the MU-1 may be fused through "linker" sequences to the Fc portion of an immunoglobulin. Other fusion proteins, such as those with GST, Lex-A or MBP, may also be used.

Also disclosed are allelic variants of the nucleotide sequence as set forth in SEQ ID NO:1 and SEQ ID NO:9, that is, naturally-occurring alternative forms of the isolated polynucleotide of SEQ ID NO:1 or SEQ ID NO:9 which also encode MU-1 proteins, preferably those proteins having a biological activity of MU-1. Also included in the invention are isolated polynucleotides which hybridize to the nucleotide sequence set forth in SEQ ID NO:1 or SEQ ID NO:9 under highly stringent conditions (for example, 0.1xSSC at 65°C). Isolated polynucleotides which encode MU-1 proteins but which differ from the nucleotide sequence set forth in SEQ ID NO: 1 or SEQ ID NO:9 by virtue of the degeneracy of the genetic code are also encompassed by the present invention. Variations in the nucleotide sequence as set forth in SEQ ID NO:1 or SEQ ID NO:9 which are caused by point mutations or by induced modifications are also included.

As used herein, the term "hybridizes under stringent conditions" is intended to describe conditions for hybridization and washing under which nucleotide sequences at least 60% identical to each other typically remain hybridized to each other. Preferably, the conditions are such that sequences at least about 70%, more preferably at least about 80%, even more preferably at least about 85% or 90% identical to each other typically remain hybridized to each other. Such stringent conditions are known to those skilled in the art and can be found in *Current Protocols in Molecular Biology,* John Wiley & Sons, N.Y. (1989), 6.3.1-6.3.6. A preferred, non-limiting example of stringent hybridization conditions are hybridization in 6X sodium chloride/sodium citrate (SSC) at about 45°C, followed by one or more washes in 0.2 X SSC, 0.1% SDS at 50°C, preferably at 55°C, more preferably at 60°C, and even more preferably at 65°C. Ranges that span the above recited values, e.g., 55-60°C, or 50-65°C are encompassed Preferably, an isolated nucleic acid molecule that hybridizes under stringent conditions to the sequence of SEQ ID NO: 1 or 9 corresponds to a naturally-occurring nucleic acid molecule. As used herein, a "naturally-occurring" nucleic acid molecule refers to an RNA or DNA molecule having a nucleotide sequence that occurs in nature (*e.g*., encodes a natural protein).

To determine the percent identity of two amino acid sequences or of two nucleic acid sequences, the sequences are aligned for optimal comparison purposes (*e.g.,* gaps can be introduced in one or both of a first and a second amino acid or nucleic acid sequence for optimal alignment and non-identical sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, or 90% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch (*J. Mol. Biol.* (48):444-453 (1970)) algorithm which has been incorporated into the GAP program in the GCG software package (available at http://www.gcg.com), using either a Blosum 62 matrix or a PAM250 matrix, and a gap weight of 16,14,12,10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. In another embodiment, the percent identity between two amino acid or nucleotide sequences is determined using the algorithm of E. Meyers and W. Miller (Myers and Miller,1988, *Comput. Appl. Biosci.* 4:11-17) which has been incorporated into the ALIGN program (version 2.0), using a PAM120 weight residue table, a gap length penalty of 12 and a gap penalty of 4.

The nucleic acid and protein sequences disclosed herein can further be used as a "query sequence" to perform a search against public databases to, for example, identify other family members or related sequences. Such searches can be performed using the NBLAST and XBLAST programs (version 2.0) of Altschul, *et al..* (1990) *J. Mol. Biol.* 215:403-10. BLAST nucleotide searches can be performed with the NBLAST program, score =100, wordlength =12 to obtain nucleotide sequences homologous to Adhr-1 nucleic acid molecules of the invention. BLAST protein searches can be performed with the XBLAST program, score =100, wordlength = 3 to obtain amino acid sequences homologous to Adhr-1 protein molecules of the invention. To obtain gapped alignments for comparison purposes, Gapped BLAST can be utilized as described in Altschul *et al..,* (1997) *Nucleic Acids Res.* 25(17):3389-3402. When utilizing BLAST and Gapped BLAST programs, the default parameters of the respective programs (*e.g.,* XBLAST and NBLASI) can be used. See http://www.ncbi.nlm.nih.gov.

Also disclosed are polynucleotides encoding homologues of the human MU-1 from other animal species, particularly other mammalian species. Species homologues can be identified and isolated by making probes or primers from the murine or human sequences disclosed herein and screening a library from an appropriate species, such as for example libraries constructed from PBMCs, thymus or testis of the relevant species.

The isolated polynucleotides may be operably linked to an expression control sequence such as the pMT2 or pED expression vectors disclosed in Kaufman et al., Nucleic Acids Res. 19,4485-4490 (1991), in order to produce the MU-1 protein recombinantly. Many suitable expression control sequences are known in the art. General methods of expressing recombinant proteins are also known and are exemplified in R. Kaufman, Methods in Enzymology 185, 537-566 (1990). As defined herein "operably linked" means enzymatically or chemically ligated to form a covalent bond between the isolated polynucleotide and the expression control sequence, in such a way that the MU-1 protein is expressed by a host cell which has been transformed (transfected) with the ligated polynucleotide/expression control sequence.

A number of types of cells may act as suitable host cells for expression of the MU-1 protein. Any cell type capable of expressing functional MU-1 protein may be used. Suitable mammalian host cells include, for example, monkey COS cells, Chinese Hamster Ovary (CHO) cells, human kidney 293 cells, human epidermal A431 cells, human Colo205 cells, 3T3 cells, CV-1 cells, other transformed primate cell lines, normal diploid cells, cell strains derived from in vitro culture of primary tissue, primary explants, HeLa cells, mouse L cells, BHK, HL-60, U937, HaK, Rat2, BaF3, 32D, FDCP-1, PC12, M1x or C2C12 cells.

The MU-1 protein may also be produced by operably linking the isolated polynucleotide to suitable control sequences in one or more insect expression vectors, and employing an insect expression system. Materials and methods for baculovirus/insect cell expression systems are commercially available in kit form from, e.g., Invitrogen, San Diego, California, U.S.A. (the MaxBac® kit), and such methods are well known in the art, as described in Summers and Smith, Texas Agricultural Experiment Station Bulletin No. 1555 (1987). Soluble forms of the MU-1 protein may also be produced in insect cells using appropriate isolated polynucleotides as described above.

Alternatively, the MU-1 protein may be produced in lower eukaryotes such as yeast or in prokaryotes such as bacteria. Suitable yeast strains include Saccharomyces cerevisiae, Schizosaccharomyces pombe, Kluyveromyces strains, *Candida,* or any yeast strain capable of expressing heterologous proteins. Suitable bacterial strains include *Escherichia coli, Bacillus subtilis, Salmonella typhimurium,* or any bacterial strain capable of expressing heterologous proteins.

Expression in bacteria may result in formation of inclusion bodies incorporating the recombinant protein. Thus, refolding of the recombinant protein may be required in order to produce active or more active material. Several methods for obtaining correctly folded heterologous proteins from bacterial inclusion bodies are known in the art These methods generally involve solubilizing the protein from the inclusion bodies, then denaturing the protein completely using a chaotropic agent. When cysteine residues are present in the primary amino acid sequence of the protein, it is often necessary to accomplish the refolding in an environment which allows correct formation of disulfide bonds (a redox system). General methods of refolding are disclosed in Kohno, Meth. Enzym., 185:187-195 (1990). EP 0433225 and copending application USSN 08/163,877 describe other appropriate methods.

The MU-1 protein may also be expressed as a product of transgenic animals, e.g., as a component of the milk of transgenic cows, goats, pigs, or sheep which are characterized by somatic or germ cells containing a polynucleotide sequence encoding the MU-1 protein.

The MU-1 protein may be prepared by growing a culture transformed host cells under culture conditions necessary to express the desired protein. The resulting expressed protein may then be purified from the culture medium or cell extracts. Soluble forms of the MU-1 protein can be purified from conditioned media. Membrane-bound forms of MU-1 protein can be purified by preparing a total membrane fraction from the expressing cell and extracting the membranes with a non-ionic detergent such as Triton X-100.

The MU-1 protein can be purified using methods known to those skilled in the art. For example, the MU-1 protein can be concentrated using a commercially available protein concentration filter, for example, an Amicon or Millipore Pellicon ultrafiltration unit. Following the concentration step, the concentrate can be applied to a purification matrix such as a gel filtration medium. Alternatively, an anion exchange resin can be employed, for example, a matrix or substrate having pendant diethylaminoethyl (DEAR) or polyethylenemine (PEI) groups. The matrices can be acrylamide, agarose, dextran, cellulose or other types commonly employed in protein purification. Alternatively, a cation exchange step can be employed. Suitable cation exchangers include various insoluble matrices comprising sulfopropyl or carboxymethyl groups. Sulfopropyl groups are preferred (e.g., S-Sepharose® columns). The purification of the MU-1 protein from culture supernatant may also include one or more column steps over such affinity resins as concanavalin A-agarose, heparin-toyopearl® or Cibacrom blue 3GA Sepharose®; or by hydrophobic interaction chromatography using such resins as phenyl ether, butyl ether, or propyl ether; or by immunoaffinty chromatography. Finally, one or more reverse-phase high performance liquid chromatography (RP-HPLC) steps employing hydrophobic RP-HPLC media, e.g., silica gel having pendant methyl or other aliphatic groups, can be employed to further purify the MU-1 protein. Affinity columns including antibodies to the MU-1 protein can also be used in purification in accordance with known methods. Some or all of the foregoing purification steps, in various combinations or with other known methods, can also be employed to provide a substantially purified isolated recombinant protein. Preferably, the isolated MU-1 protein is purified so that it is substantially free of other mammalian proteins.

MU-1 proteins may also be used to screen for agents which are capable of binding to MU-1. Binding assays using a desired binding protein, immobilized or not, are well known in the art and may be used for this purpose using the MU-1 protein of the invention. Purified cell based or protein based (cell free) screening assays may be used to identify such agents. For example, MU-1 protein may be immobilized in purified form on a carrier and binding or potential ligands to purified. MU-1 protein may be measured.

MU-1 proteins, purified from cells or recombinantly produced, may be used as a pharmaceutical composition when combined with a pharmaceutically acceptable carrier. Such a composition may contain, in addition to MU-1 or inhibitor and carrier, various diluents, fillers, salts, buffers, stabilizers, solubilizers, and other materials well known in the art. The term "pharmaceutically acceptable" means a non-toxic material that does not interfere with the effectiveness of the biological activity of the active ingredient(s). The characteristics of the carrier will depend on the route of administration.

The pharmaceutical composition may also contain cytokines, lymphokines, or other hematopoietic factors such as M-CSF, OM-CSF, IL-1, IL-2, IL-3, IL-4, IL-5, IL-6, IL-7, IL-8, IL-9, IL-10, IL-11, IL-12, IL-14, IL-15, G-CSF, stem cell factor, and erythropoietin. The pharmaceutical composition may also include anti-cytokine antibodies. The pharmaceutical composition may contain thrombolytic or anti-thrombotic factors such as plasminogen activator and Factor VIII. The pharmaceutical composition may further contain other anti-inflammatory agents. Such additional factors and/or agents may be included in the pharmaceutical composition to produce a synergistic effect with isolated MU-1 protein, or to minimize side effects caused by the isolated MU-1 protein. Conversely, isolated MU-1 protein may be included in formulations of the particular cytokine, lymphokine, other hematopoietic factor, thrombolytic or anti-thrombotic factor, or anti-inflammatory agent to minimize side effects of the cytokine, lymphokine, other hematopoietic factor, thrombolytic or anti-thrombotic factor, or anti-inflammatory agent.

The pharmaceutical composition may be in the form of a liposome in which isolated MU-1 protein is combined, in addition to other pharmaceutically acceptable carriers, with amphipathic agents such as lipids which exist in aggregated form as micelles, insoluble monolayers, liquid crystals, or lamellar layers. Suitable lipids for liposomal formulation include, without limitation, monoglycerides, diglycerides, sulfatides, lysolecithin, phospholipids, saponin, bile acids, and the like. Preparation of such liposomal formulations is within the level of skill in the art, as disclosed, for example, in U.S. Patent No. 4,235,871; U.S. Patent No. 4,501,728; U.S. Patent No. 4,837,028; and U.S. Patent No. 4,737,323.

As used herein, the term "therapeutically effective amount" means the total amount of each active component of the pharmaceutical composition or method that is sufficient to show a meaningful patient benefit, e.g., amelioration of symptoms of, healing of, or increase in rate of healing of such conditions. When applied to an individual active ingredient, administered alone, the term refers to that ingredient alone. When applied to a combination, the term refers to combined amounts of the active ingredients that result in the therapeutic effect, whether administered in combination, serially or simultaneously.

Isolated MU-1 protein may be administered either alone or in combination with other therapies such as treatments employing cytokines, lymphokines or other hematopoietic factors. When co-administered with one or more cytokines, lymphokines or other hematopoietic factors, MU-1 protein may be administered either simultaneously with the cytokine(s), lymphokine(s), other hematopoietic factor(s), thrombolytic or anti-thrombotic factors, or sequentially. If administered sequentially, the attending physician will decide on the appropriate sequence of administering MU-1 protein in combination with cytokine(s), lymphokine(s), other hematopoietic factor(s), thrombolytic or anti-thmmbotic factors.

Administration of MU-1 protein used in the pharmaceutical composition or to practice the method of the present invention can be carried out in a variety of conventional ways, such as oral ingestion, inhalation, or cutaneous, subcutaneous, or intravenous injection. Intravenous administration to the patient is preferred.

When a therapeutically effective amount of MU-1 protein is administered orally, MU-1 protein will be in the form of a tablet, capsule, powder, solution or elixir. When administered in tablet form, the pharmaceutical composition of the invention may additionally contain a solid carrier such as a gelatin or an adjuvant. The tablet, capsule, and powder contain from about 5 to 95% MU-1 protein, and preferably from about 25 to 90% MU-1 protein. When administered in liquid form, a liquid carrier such as water, petroleum, oils of animal or plant origin such as peanut oil, mineral oil, soybean oil, or sesame oil, or synthetic oils may be added. The liquid form of the pharmaceutical composition may further contain physiological saline solution, dextrose or other saccharide solution, or glycols such as ethylene glycol, propylene glycol or polyethylene glycol. When administered in liquid form, the pharmaceutical composition contains from about 0.5 to 90% by weight of MU-1 protein, and preferably from about 1 to 50% MU-1 protein.

When a therapeutically effective amount of ML1-1 protein is administered by intravenous, cutaneous or subcutaneous injection, MU-1 protein will be in the form of a pyrogen-free, parenterally acceptable aqueous solution. The preparation of such parenterally acceptable protein solutions, having due regard to pH, isotonicity, stability, and the like, is within the skill in the art. A preferred pharmaceutical composition for intravenous, cutaneous, or subcutaneous injection should contain, in addition to MU-1 protein an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical composition may also contain stabilizers, preservatives, buffers, antioxidants, or other additive known to those of skill in the art.

The amount of MU-1 protein in the pharmaceutical composition will depend upon the nature and severity of the condition being treated, and on the nature of prior treatments which the patient has undergone. Ultimately, the attending physician will decide the amount of MU-1 protein with which to treat each individual patient Initially, the attending physician will administer low doses of MU-1 protein and observe the patient's response. Larger doses of MU-1 protein may be administered until the optimal therapeutic effect is obtained for the patient, and at that point the dosage is not generally increased further. It is contemplated that the various pharmaceutical compositions used should contain about 0.1 µg to about 100 mg of MU-1 protein per kg body weight

The duration of intravenous therapy using the pharmaceutical composition will vary, depending on the severity of the disease being treated and the condition and potential idiosyncratic response of each individual patient. It is contemplated that the duration of each application of the MU-1 protein will be in the range of 12 to 24 hours of continuous intravenous administration. Ultimately the attending physician will decide on the appropriate duration of intravenous therapy using the pharmaceutical composition.

The polynucleotide and proteins are expected to exhibit one or more of the uses or biological activities (including those associated with assays cited herein) identified below. Uses or activities described for proteins may be provided by administration or use of such proteins or by administration or use of polynucleotides encoding such proteins (such as, for example, in gene therapies or vectors suitable for introduction of DNA).

### Cytokine and Cell Proliferation/Differentiation Activity

A MU-1 protein may exhibit cytokine, cell proliferation (either inducing or inhibiting) or cell differentiation (either inducing or inhibiting) activity or may induce production of other cytokines in certain cell populations.

Many protein factors discovered to date, including all known cytokines, have exhibited activity in one or more factor dependent cell proliferation assays, and hence the assays serve as a convenient confirmation of cytokine activity. The activity of a MU-1 protein is evidenced by any one of a number of routine factor dependent cell proliferation assays for cell lines including, without limitation, 32D, DA2, DA1G, T10, B9, B9/11, BaF3, MC9/G, M+ (preB M+), 2E8, RB5, DA1,123, T1 165, HT2, CTLL2, TF-1, Mo7e and CMK.

BAF-3 cells expressing huEPOR -huMU chimeric receptors proliferate in response to huEPO. In order to test the capacity for the MU receptor to signal through their cytoplasmic domain, BAF-3 cells were engineered to express EPOr/MU(cyto) chimeric receptors and assayed for ³H thymidine incorporation in the presence of EPO. BAF-3 cells expressing intact EPOr molecules proliferate in response to EPO while parent BAF-3 cells do not. The A5 clone, possessing the chimeric EPOr/MU(cyto) proliferates in response to EPO demonstrating that the cytoplasmic portion of MU-1 can sustain a proliferative signal. The BAF-3 cells which express EPOr on their surface also respond to EPO.

The activity of a protein may, among other means, be measured by the following methods:

Assays for T-cell or thymocyte proliferation include without limitation those described in: Current Protocols in Immunology, Ed by J. E. Coligan, A.M. Kruisbeek, D.H. Margulies, E.M. Shevach, W Strober, Pub. Greene Publishing Associates and Wiley-Interscience (Chapter 3, In Vitro assays for Mouse Lymphocyte Function 3.1-3.19; Chapter 7, Immunologic studies in Humans); Takai et al., J. Immunol. 137:3494-3500, 1986; Bertagnolli et al., J. Immunol. 145:1706-1712, 1990; Bertagnolli et al., Cellular Immunology 133:327-341, 1991; Bertagnolli, et al., J. Immunol. 149:3778-3783, 1992; Bowman et al., J. Immunol. 152: 1756-1761, 1994.

Assays for cytokine production and/or proliferation of spleen cells, lymph node cells or thymocytes include, without limitation, those described in: Polyclonal T cell stimulation, Kruisbeek, A.M. and Shevach, E.M. In *Current Protocols in Immunology.* J.E.e.a. Coligan eds. Vol 1 pp. 3.12.1-3.12.14, John Wiley and Sons, Toronto. 1994; and Measurement of mouse and human Interferon γ, Schreiber, R.D. In *Current Protocols in Immunology.* J.E.e.a. Coligan eds. Vol 1 pp. 6.8.1-6.8.8, John Wiley and Sons, Toronto. 1994.

Assays for proliferation and differentiation of hematopoietic and lymphopoietic cells include, without limitation, those described in: Measurement of Human and Murine Interleukin 2 and Interleukin 4, Bottomly, K., Davis, L.S. and Lipsky, P.E. In *Current Protocols in Immunology.* J.E.e.a. Coligan eds. Vol 1 pp. 6.3.1-6.3.12, John Wiley and Sons, Toronto. 1991; deVries et al., J. Exp. Med. 173:1205-1211, 1991; Moreau et al., Nature 336:690-692, 1988; Greenberger et al., Proc. Natl. Acad. Sci. U.S.A. 80:2931-2938, 1983; Measurement of mouse and human interleukin 6 - Nordan, R. In *Current Protocols in Immunology.* J.E.e.a. Coligan eds. Vol 1 pp. 6.6.1-6.6.5, John Wiley and Sons, Toronto. 1991; Smith et al., Proc. Natl. Acad. Sci. U.S.A. 83:1857-1861, 1986; Measurement of human Interleukin 11 - Bennett, F., Giannotti, J., Clark, S.C. and Turner, K. J. In *Current Protocols in Immunology.* J.E.e.a. Coligan eds. Vol 1 pp. 6.15.1 John Wiley and Sons, Toronto. 1991; Measurement of mouse and human Interleukin 9 - Ciarletta, A., Giannotti, J., Clark, S.C. and Turner, K.J. In *Current Protocols in Immunology.* J.E.e.a. Coligan eds. Vol 1 pp. 6.13.1, John Wiley and Sons, Toronto. 1991.

Assays for T-cell clone responses to antigens (which will identify, among others, proteins that affect APC-T cell interactions as well as direct T-cell effects by measuring proliferation and cytokine production) include, without limitation, those described in: Current Protocols in Immunology, Ed by J. E. Coligan, A.M. Kruisbeek, D.H. Margulies, E.M. Shevach, W Strober, Pub. Greene Publishing Associates and Wiley-Interscience (Chapter 3, In Vitro assays for Mouse Lymphocyte Function; Chapter 6, Cytokines and their cellular receptors; Chapter 7, Immunologic studies in Humans); Weinberger et al., Proc. Natl. Acad. Sci. USA 77:6091-6095,1980; Weinberger et al., Eur. J. Immnn.11:405-411, 1981; Takai et al., J. Immunol. 137:3494-3500,1986; Takai et al., J. Immunol. 140:508-512, 1988.

### Immune Stimulating or Suppressing Activity

A MU-1 protein may also exhibit immune stimulating or immune suppressing activity, including without limitation the activities for which assays are described herein. A protein may be useful in the treatment of various immune deficiencies and disorders (including severe combined immunodeficiency (SCID)), e.g., in regulating (up or down) growth and proliferation of T and/or B lymphocytes, as well as effecting the cytolytic activity ofNK cells and other cell populations. These immune deficiencies may be genetic or be caused by viral (e.g., HIV) as well as bacterial or fungal infections, or may result from autoimmune disorders. More specifically, infectious diseases causes by viral, bacterial, fungal or other infection may be treatable using a protein of the present invention, including infections by HIV, hepatitis viruses, herpesviruses, mycobacteria, Leishmania spp., malaria spp. and various fungal infections such as candidiasis. Of course, in this regard, a disclosed protein may also be useful where a boost to the immune system generally may be desirable, *i.e.,* in the treatment of cancer.

Autoimmune disorders which may be treated using a disclosed protein include, for example, connective tissue disease, multiple sclerosis, systemic lupus erythematosus, rheumatoid arthritis, autoimmune pulmonary inflammation, Guillain-Barre syndrome, autoimmune thyroiditis, insulin dependent diabetes mellitus, myasthenia gravis, graft-versus-host disease and autoimmune inflammatory eye disease. Such a protein may also to be useful in the treatment of allergic reactions and conditions, such as asthma (particularly allergic asthma) or other respiratory problems. Other conditions, in which immune suppression is desired (including, for example, organ transplantation), may also be treatable using a disclosed protein.

The MU-1 DNA also maps to the chromosomal locus for Crohn's disease. As a result, MU-1 proteins may be used to treat Crohn's disease and other inflammatory bowel diseases.

Using the MU-1 proteins disclosed herein it may also be possible to regulate immune responses, in a number of ways. Down regulation may be in the form of inhibiting or blocking an immune response already in progress or may involve preventing the induction of an immune response. The functions of activated T cells may be inhibited by suppressing T cell responses or by inducing specific tolerance in T cells, or both. Immunosuppression of T cell responses is generally an active, non-antigen-specific, process which requires continuous exposure of the T cells to the suppressive agent. Tolerance, which involves inducing non-responsiveness or anergy in T cells, is distinguishable from immunosuppression in that it is generally antigen-specific and persists after exposure to the tolerizing agent has ceased. Operationally, tolerance can be demonstrated by the lack of a T cell response upon reexposure to specific antigen in the absence of the tolerizing agent.

Down regulating or preventing one or more antigen functions (including without limitation B lymphocyte antigen functions (such as, for example, B7)), e.g., preventing high level lymphokine synthesis by activated T cells, will be useful in situations of tissue, skin and organ transplantation and in graft-versus-host disease (GVHD). For example, blockage ofT cell function should result in reduced tissue destruction in tissue transplantation. Typically, in tissue transplants, rejection of the transplant is initiated through its recognition as foreign by T cells, followed by an immune reaction that destroys the transplant. The administration of a molecule which inhibits or blocks interaction of a B7 lymphocyte antigen with its natural ligand(s) on immune cells (such as a soluble, monomeric form of a peptide having B7-2 activity alone or in conjunction with a monomeric form ofa peptide having an activity of another B lymphocyte antigen (e.g., B7-1, B7-3) or blocking antibody), prior to transplantation can lead to the binding of the molecule to the natural ligand(s) on the immune cells without transmitting the corresponding costimulatory signal. Blocking B lymphocyte antigen function in this matter prevents cytokine synthesis by immune cells, such as T cells, and thus acts as an immunosuppressant. Moreover, the lack of costimulation may also be sufficient to anergize the T cells, thereby inducing tolerance in a subject. Induction of long-term tolerance by B lymphocyte antigen-blocking reagents may avoid the necessity of repeated administration of these blocking reagents. To achieve sufficient immunosuppression or tolerance in a subject, it may also be necessary to block the function of a combination of B lymphocyte antigens.

The efficacy of particular blocking reagents in preventing organ transplant rejection or GVHD can be assessed using animal models that are predictive of efficacy in humans. Examples of appropriate systems which can be used include allogeneic cardiac grafts in rats and xenogeneic pancreatic islet cell grafts in mice, both of which have been used to examine the immunosuppressive effects of CTLA4Ig fusion proteins *in vivo* as described in Lenschow *et al.,* Science *257*:789-792 (1992) and Turka *et al.,* Proc. Natl. Acad. Sci USA, 89:11102-11105 (1992). In addition, murine models of GVHD (see Paul ed., Fundamental Immunology, Raven Press, New York, 1989, pp. 846-847) can be used to determine the effect of blocking B lymphocyte antigen function *in vivo* on the development of that disease.

Blocking antigen function may also be therapeutically useful for treating autoimmune diseases. Many autoimmune disorders are the result of inappropriate activation of T cells that are reactive against self tissue and which promote the production of cytokines and autoantibodies involved in the pathology of the diseases. Preventing the activation of autoreactive T cells may reduce or eliminate disease symptoms. Administration of reagents which block costimulation of T cells by disrupting receptor:ligand interactions of B lymphocyte antigens can be used to inhibit T cell activation and prevent production of autoantibodies or T cell-derived cytokines which may be involved in the disease process. Additionally, blocking reagents may induce antigen-specific tolerance of autoreactive T cells which could lead to long-term relief from the disease. The efficacy of blocking reagents in preventing or alleviating autoimmune disorders can be determined using a number of well-characterized animal models of human autoimmune diseases. Examples include murine experimental autoimmune encephalitis, systemic lupus erythmatosis in MRL/*lpr*/*lpr* mice or NZB hybrid mice, murine autoimmune collagen arthritis, diabetes mellitus in NOD mice and BB rats, and murine experimental myasthenia gravis (see Paul ed., Fundamental Immunology, Raven Press, New York, 1989, pp. 840-856).

Upregulation of an antigen function (preferably a B lymphocyte antigen function), as a means of up regulating immune responses, may also be useful in therapy. Upregulation of immune responses may be in the form of enhancing an existing immune response or eliciting an initial immune response. For example, enhancing an immune response through stimulating B lymphocyte antigen function may be useful in cases of viral infection. In addition, systemic viral diseases such as influenza, the common cold, and encephalitis might be alleviated by the administration of stimulatory forms of B lymphocyte antigens systemically.

Alternatively, anti-viral immune responses may be enhanced in an infected patient by removing T cells from the patient, costimulating the T cells *in vitro* with viral antigen-pulsed APCs either expressing a MU-1 peptide or together with a stimulatory form of a soluble peptide disclosed herein and reintroducing the *in vitro* activated T cells into the patient Another method of enhancing anti-viral immune responses would be to isolate infected cells from a patient, transfect them with a nucleic acid encoding a protein as described herein such that the cells express all or a portion of the protein on their surface, and reintroduce the transfected cells into the patient The infected cells would now be capable of delivering a costimulatory signal to, and thereby activate, T cells *in vivo.*

In another application, up regulation or enhancement of antigen function (preferably B lymphocyte antigen function) may be useful in the induction of tumor immunity. Tumor cells (e.g., sarcoma, melanoma, lymphoma, leukemia, neuroblastoma, carcinoma) transfected with a nucleic acid encoding at least one peptide of the present invention can be administered to a subject to overcome tumor-specific tolerance in the subject. If desired, the tumor cell can be transfected to express a combination of peptides. For example, tumor cells obtained from a patient can be transfected *ex vivo* with an expression vector directing the expression of a peptide having B7-2-like activity alone, or in conjunction with a peptide having B7-1-like activity and/or B7-3-like activity. The transfected tumor cells are returned to the patient to result in expression of the peptides on the surface of the transfected cell. Alternatively, gene therapy techniques can be used to target a tumor cell for transfection *in vivo.*

The presence of the disclosed peptide having the activity of a B lymphocyte antigen(s) on the surface of the tumor cell provides the necessary costimulation signal to T cells to induce a T cell mediated immune response against the transfected tumor cells. In addition, tumor cells which lack MHC class I or MHC class II molecules, or which fail to reexpress sufficient amounts of MHC class I or MHC class II molecules, can be transfected with nucleic acid encoding all or a portion of (*e.g.,* a cytoplasmic-domain truncated portion) of an MHC class I α chain protein and β₂ microglobulin protein or an MHC class II α chain protein and an MHC class II β chain protein to thereby express MHC class I or MHC class II proteins on the cell surface. Expression of the appropriate class I or class II MHC in conjunction with a peptide having the activity of a B lymphocyte antigen (*e.g.,* B7-1, B7-2, B7-3) induces a T cell mediated immune response against the transfected tumor cell. Optionally, a gene encoding an antisense construct which blocks expression of an MHC class II associated protein, such as the invariant chain, can also be cotransfected with a DNA encoding a peptide having the activity of a B lymphocyte antigen to promote presentation of tumor associated antigens and induce tumor specific immunity. Thus, the induction of a T cell mediated immune response in a human subject may be sufficient to overcome tumor-specific tolerance in the subject

The activity of a disclosed protein may, among other means, be measured by the following methods:

Suitable assays for thymocyte or splenocyte cytotoxicity include, without limitation, those described in: Current Protocols in Immunology, Ed by J. E. Coligan, A.M. Kruisbeek, D.H. Margulies, E.M. Shevach, W Strober, Pub. Greene Publishing Associates and Wiley-Interscience (Chapter 3, In Vitro assays for Mouse Lymphocyte Function 3.1-3.19; Chapter 7, Immunologic studies in Humans); Herrmann et al., Proc. Natl. Acad. Sci. USA 78:2488-2492, 1981; Herrmann et al., J. Immunol. 128:1968-1974, 1982; Handa et al., J. Immunol. 135:1564-1572, 1985; Takai et al., J. Immunol. 137:3494-3500, 1986; Takai et al., J. Immunol. 140:508-512, 1988; Herrmann et al., Proc. Natl. Acad. Sci. USA 78:2488-2492,1981; Herrmann et al., J. Immunol. 128:1968-1974, 1982; Handa et al., J. Immunol. 135:1564-1572, 1985; Takai et al., J. Immunol. 137:3494-3500, 1986; Bowmanet al., J. Virology 61:1992-1998; Takai et al., J. Immunol. 140:508-512, 1988; Bertagnolli et al., Cellular Immunology 133:327-341, 1991; Brown et al., J. Immunol. 153:3079-3092, 1994.

Assays for T-cell-dependent immunoglobulin responses and isotype switching (which will identify, among others, proteins that modulate T-cell dependent antibody responses and that affect Th1/Th2 profiles) include, without limitation, those described in: Maliszewski, J. Immunol. 144:3028-3033, 1990; and Assays for B cell function: *In vitro* antibody production, Mond, J.J. and Brunswick, M. In *Current Protocols in Immunology.* J.E.e.a. Coligan eds. Vol 1 pp. 3.8.1-3.8.16, John Wiley and Sons, Toronto. 1994.

Mixed lymphocyte reaction (MLR) assays (which will identify, among others, proteins that generate predominantly Th1 and CTL responses) include, without limitation, those described in: Current Protocols in Immunology, Ed by J. E. Coligan, A.M. Kruisbeek, D.H. Margulies, E.M. Shevach, W Strober, Pub. Greene Publishing Associates and Wiley-Interscience (Chapter 3, In Vitro assays for Mouse Lymphocyte Function 3.1-3.19; Chapter 7, Immunologic studies in Humans); Takai et al., J. Immunol. 137:3494-3500, 1986; Takai et al., J. Immunol. 140:508-512, 1988; Bertagnolli et al., J. Immunol. 149:3778-3783, 1992.

Dendritic cell-dependent assays (which will identify, among others, proteins expressed by dendritic cells that activate naive T-cells) include, without limitation, those described in: Guery et al., J. Immunol. 134:536-544, 1995; Inaba et al., Journal of Experimental Medicine 173:549-559, 1991; Macatonia et al., Journal of Immunology 154:5071-5079, 1995; Porgador et al., Journal of Experimental Medicine 182:255-260, 1995; Nair et al., Journal of Virology 67:4062-4069, 1993; Huang et al., Science 264:961-965, 1994; Macatonia et al., Journal of Experimental Medicine 169:1255-1264, 1989; Bhardwaj et al., Journal of Clinical Investigation 94:797-807, 1994; and Inaba et al., Journal of Experimental Medicine 172:631-640, 1990.

Assays for lymphocyte survival/apoptosis (which will identify, among others, proteins that prevent apoptosis after superantigen induction and proteins that regulate lymphocyte homeostasis) include, without limitation, those described in: Darzynkiewicz et al., Cytometry 13:795-808, 1992; Gorczyca et al., Leukemia 7:659-670,1993; Gorczyca et al., Cancer Research 53:1945-1951,1993; Itoh et al., Cell 66:233-243, 1991; Zacharchuk, Journal of Immunology 145:4037-4045,1990; Zamai et al., Cytometry 14:891-897, 1993; Gorczyca et al., International Journal of Oncology 1:639-648,1992.

Assays for proteins that influence early steps of T-cell commitment and development include, without limitation, those described in: Antica et aL, Blood 84:111-117, 1994; Fine et al., Cellular Immunology 155:111-122,1994; Galy et al., Blood 85:2770-2778,1995; Toki et al., Proc. Nat. Acad Sci. USA 88:7548-7551, 1991.

The MCT-1 molecules of the present invention are also involved in the modulation of STAT activity, *e.g.,* signaling (*e.g*., through interaction with STAT molecules, e.g., STAT 3 and/or STAT 5, and modulation of STAT phosphorylation). Modulators of STAT signaling and modulators of MU-1 interaction with STAT molecules can be identified through screening assays as described herein.

Assays that may be used to identify modulators of MU-1 activity include assays for STAT activity, e.g., STAT signaling, such as assays known in the art and assays described herein. Assays for STAT activity are also described in, for example, Friedrich K, *et al.* (2001) *Biol Chem* 382(2):343-51. Other assays for STAT activity include assays for cell proliferation and STAT phosphorylation.

In one aspect of the invention, an assay for a modulator of MU-1 activity is a cell-based assay in which a cell which expresses a MU-1 protein or biologically active portion thereof is contacted with a test compound and the ability of the test compound to modulate MU-1 activity (*e.g.,* interaction with STAT molecules or modulation of STAT signaling) is determined. Determining the ability of the test compound to modulate MU-1 activity can be accomplished by monitoring, for example, STAT signaling, phosphorylation of STAT molecules, cell proliferation, cell differentiation, or production of cytokines. The cell, for example, can be of mammalian origin, *e.g.,* an activated T-cell.

### Hematopoiesis Regulating Activity

A MU-1 protein may be useful in regulation of hematopoiesis and, consequently, in the treatment of myeloid or lymphoid cell deficiencies. Even marginal biological activity in support of colony forming cells or of factor-dependent cell lines indicates involvement in regulating hematopoiesis, e.g. in supporting the growth and proliferation of erythroid progenitor cells alone or in combination with other cytokines, thereby indicating utility, for example, in treating various anemias or for use in conjunction with irradiation/chemotherapy to stimulate the production of erythroid precursors and/or erythroid cells; in supporting the growth and proliferation of myeloid cells such as granulocytes and monocytes/macrophages (i.e., traditional CSF activity) useful, for example, in conjunction with chemotherapy to prevent or treat consequent myelo-suppression; in supporting the growth and proliferation of megakaryocytes and consequently of platelets thereby allowing prevention or treatment of various platelet disorders such as thrombocytopenia, and generally for use in place of or complimentary to platelet transfusions; and/or in supporting the growth and proliferation of hematopoietic stem cells which are capable of maturing to any and all of the above-mentioned hematopoietic cells and therefore find therapeutic utility in various stem cell disorders (such as those usually treated with transplantation, including, without limitation, aplastic anemia and paroxysmal nocturnal hemoglobinuria), as well as in repopulating the stem cell compartment post irradiation/chemotherapy, either *in-vivo* or *ex-vivo* (i.e., in conjunction with bone marrow transplantation or with peripheral progenitor cell transplantation (homologous or heterologous)) as normal cells or genetically manipulated for gene therapy.

The activity of a MU-1 protein may, among other means, be measured by the following methods:

Suitable assays for proliferation and differentiation of various hematopoietic lines are cited above.

Assays for embryonic stem cell differentiation (which will identify, among others, proteins that influence embryonic differentiation hematopoiesis) include, without limitation, those described in: Johansson et al. Cellular Biology 15:141-151,1995; Keller et al., Molecular and Cellular Biology 13:473-486,1993; McClanahan et al., Blood 81:2903-2915, 1993.

Assays for stem cell survival and differentiation (which will identify, among others, proteins that regulate lympho-hematopoiesis) include, without limitation, those described in: Methylcellulose colony forming assays, Freshney, M.G. In *Culture of Hematopoietic Cells.* RI. Freshney, *et al.* eds. Vol pp. 265-268, Wiley-Liss, Inc., New York, NY. 1994; Hirayama et al., Proc. Natl. Acad: Sci. USA 89:5907-5911,1992; Primitive hematopoietic colony forming cells with high proliferative potential, McNiece, I.K. and Briddell, R.A. In *Culture of Hematopoietic Cells.* RI. Freshney, *et al.* eds. Vol pp. 23-39, Wiley-Liss, Inc., New York, NY. 1994; Neben et al., Experimental Hematology 22:353-359,1994; Cobblestone area forming cell assay, Ploemacher, R.E. In *Culture of Hematopoietic Cells.* R.I. Freshney, *et al.* eds. Vol pp. 1-21, Wiley-Liss, Inc.., New York, NY. 1994; Long term bone marrow cultures in the presence of stromal cells, Spooncer, E., Dexter, M. and Allen, T. In *Culture of Hematopoietic Cells.* R.I. Freshney, *et al.* eds. Vol pp. 163-179, Wiley-Liss, Inc., New York, NY. 1994; Long term culture initiating cell assay, Sutherland, HJ. In *Culture of Hematopoietic Cells.* R.I. Freshney, *et al.* eds. Vol pp. 139-162, Wiley-Liss, Inc., New York, NY. 1994.

### Research Uses and Utilities

Polynucleotides disclosed herein can be used by the research community for various purposes. The polynucleotides can be used to express recombinant protein for analysis, characterization or therapeutic use; as markers for tissues in which the corresponding protein is preferentially expressed (either constitutively or at a particular stage of tissue differentiation or development or in disease states); as molecular weight markers on Southern gels; as chromosome markers or tags (when labeled) to identify chromosomes or to map related gene positions; to compare with endogenous DNA sequences in patients to identify potential genetic disorders; as probes to hybridize and thus discover novel, related DNA sequences; as a source of information to derive PCR primers for genetic fingerprinting; as a probe to "subtract-out" known sequences in the process of discovering other novel polynucleotides; for selecting and making oligomers for attachment to a "gene chip" or other support, including for examination of expression patterns; to raise anti-protein antibodies using DNA immunization techniques; and as an antigen to raise anti-DNA antibodies or elicit another immune response. Where the polynucleotide encodes a protein which binds or potentially binds to another protein (such as, for examples, in a receptor-ligand interaction), the polynucleotide can also be used in interaction trap assays (such as, for example, that described in Gyuris et al., Cell 75:791-803 (1993)) to identify polynucleotides encoding the other protein with which binding occurs or to identify inhibitors of the binding interaction.

The proteins disclosed herein can similarly be used in assays to determine biological activity, including in a panel of multiple proteins for high-throughput screening; to raise antibodies or to elicit another immune response; as a reagent (including the labeled reagent) in assays designed to quantitatively determine levels of the protein (or its receptor) in biological fluids; as markers for tissues in which the corresponding protein is preferentially expressed (either constitutively or at a particular stage of tissue differentiation or development or in a disease state); and, of course, to isolate correlative receptors or ligands. Where the protein binds or potentially binds to another protein (such as, for example, in a receptor-ligand interaction), the protein can be used to identify the other protein with which binding occurs or to identify inhibitors of the binding interaction. Proteins involved in these binding interactions can also be used to screen for peptide or small molecule inhibitors or agonists of the binding interaction.

Any or all of these research utilities are capable of being developed into reagent grade or kit format for commercialization as research products.

Methods for performing the uses listed above are well known to those skilled in the art. References disclosing such methods include without limitation "Molecular Cloning: A Laboratory Manual", 2d ed., Cold Spring Harbor Laboratory Press, Sambrook, J., E.F. Fritsch and T. Maniatis eds., 1989, and "Methods in Enzymology: Guide to Molecular Cloning Techniques", Academic Press, Berger, S.L. and A.R Kimmel eds., 1987.

### Nutritional Uses

Polynucleotides and proteins disclosed herein can also be used as nutritional sources or supplements. Such uses include without limitation use as a protein or amino acid supplement, use as a carbon source, use as a nitrogen source and use as a source of carbohydrate. In such cases the protein or polynucleotide of the invention can be added to the feed of a particular organism or can be administered as a separate solid or liquid preparation, such as in the form of powder, pills, solutions, suspensions or capsules. In the case of microorganisms, the protein or polynucleotide can be added to the medium in or on which the microorganism is cultured.

MU-1 proteins may also be used to immunize animals to obtain polyclonal and monoclonal antibodies which specifically react with the MU-1 protein and which may inhibit binding of ligands to the receptor. Such antibodies may be obtained using the entire MU-1 as an immunogen, or by using fragments of MU-1. Smaller fragments of the MU-1 may also be used to immunize animals. The peptide immunogens additionally may contain a cysteine residue at the carboxyl terminus, and are conjugated to a hapten such as keyhole limpet hemocyanin (KLH). Additional peptide immunogens may be generated by replacing tyrosine residues with sulfated tyrosine residues. Methods for synthesizing such peptides are known in the art, for example, as in RP. Merrifield, J.Amer.Chem.Soc. 85, 2149-2154 (1963); J.L. Krstenansky, et al., FEBS Lett. 211, 10 (1987).

Neutralizing or non-neutralizing antibodies (preferably monoclonal antibodies) binding to MU-1 protein may also be useful therapeutics for certain tumors and also in the treatment of conditions described above. These neutralizing monoclonal antibodies may be capable of blocking ligand binding to the MU-1 receptor chain.

This invention is further illustrated by the following examples which should not be construed as limiting.

### EXAMPLES

### Example 1: Isolation and Characterization of Murine MU-1 cDNAs:

A partial fragment of the murine homolog of the Mu-1 receptor was isolated by PCR using oligonucleotides derived from the human sequence. cDNA was prepared from RNA isolated from 17 day old murine thymus and from the murine 2D6 T cell line. A DNA fragment of approximately 300 nucleotides was amplified from the cDNA by PCR with the following oligonucleotides, corresponding to regions 584-603 and 876-896, respectively, of the human cDNA sequence in figure 1 (corresponding to SEQ ID NO:1):
AGCATCAAGC CGGCTCCCCC (5p) (SEQ ID NO:11)
CTCCATTCAC TCCAGGTCCC (3p) (SEQ ID NO:12)
Amplification was carried out using Taq polymerase in 1 x Taq buffer containing 1.5 mM of magnesium chloride for 30 cycles at 94° C for one minute, 50° C for 1 minute, and 72° C for one minute. The DNA sequence of this fragment was determined, and two oligonucleotides were derived from an internal portion of this fragment with the following sequences:
TTGAACGTGACTGTGGCCTT (5p) (SEQ ID NO:13)
TGAATGAAGTGCCTGGCTGA (3p) (SEQ ID NO:14)
The oligonucleotides were used to amplify an internal 262 nucleotide fragment of the original PCR product (corresponding to nucleotides 781-1043 in of the murine cDNA sequence of figure 1, and SEQ ID NO:9) to use as a hybridization probe to screen a cDNA library isolated from the 2D6 T cell line. Filters were hybridized at 65° C using standard 5 X SSC hybridization conditions and washed into SSC at 65° C. Twenty clones were isolated that hybridized to the probe in a screen of 426,000 clones. DNA sequence was determined from two independent clones. Full length sequence of clone #6 confirmed that it was the full-length murine homolog of human MU-1 (SEQ ID NO:9).

The full-length nucleotide sequence of murine MU-1 is shown in figure 1 (corresponding to SEQ ID NO:9). The nucleotide sequence has a predicted leader sequence at nucleotides 407-464, coding sequence at nucleotides 407-1993, termination codon at nucleotides 1994-1997. Nucleotides 1-406 correspond to the 5' untranslated region and nucleotides 1998-2628 correspond to the 3' untranslated region.

The predicted protein sequence of murine MU-1 is shown in figure 2 (corresponding to SEQ ID NO:10). This murine MU-1 protein contains a predicted leader sequence determined by SPScan (score=10.1) (corresponding to amino acids 1-19 of SEQ ID NO:10), and a predicted transmembrane domain (corresponding to amino acids 237-253 of SEQ ID NO:10). Predicted signaling motifs include the following regions: Box 1: amino acids 265-274 of SEQ ID NO:10, Box 2: amino acids 310-324 of SEQ ID NO:10, six tyrosine residues at positions 281, 319, 361, 368, 397, and 510 of SEQ ID NO:10. Potential STAT docking sites include: STATS: EDDGYPA, STAT 3:YLQR.

### Example 2: Comparison of Human and Murine MU-1:

The GAP algorithm was used to compare the human and murine MU-1 amino acids. A comparison of the murine and human predicted protein sequences is shown in figure 4. The amino acids were 65.267% identical using the GAP algorithm. The alignment was generated by BLOSUM62 amino acid substitution matrix (Henikoff, S. and Henikoff, J. G. (1992)). Amino acid substitution matrices from protein blocks (Proc. Natl. Acad. Sci. USA 89: 10915-10919). Gap parameters= Gap Weight: 8, Average Match= 2.912, Length Weight= 2, Average Mismatch= -2.003. Percent Similarity= 69.466.

A comparison of the human and murine cDNA nucleotide sequences is shown in figure 3. The DNA sequences are 66.116% identical when aligned using the GAP algorithm. Gap Parameters: Gap Weight= 50, Average Match= 10.000, Length Weight= 3, Average Mismatch= 0.000. Percent Similarity= 66.198.

Both human and mouse MU-1 proteins are members of the Type 1 cytokine receptor superfamily. Evaluation of the sequence of both murine and human MU-1 reveals the presence of potential Box-1 and Box-2 signaling motifs. Six tyrosine residues are present in the cytoplasmic domain, and could also be important in signaling functions of MU-1. Comparison of the sequences of MU-1 with other members of the family suggested the presence of potential docking sites for STAT 5 and STAT 3.

### Example 3: Determination of STAT signaling pathways used by Human MU-1:

BAF-3 cells were engineered to express a chimeric cytokine receptor consisting of the extracellular domain of the human EPO receptor and the intracellular domain of the MU-1 receptor. BAF-3 cells that expressed huEPOR/MU-1(cyto) chimeric receptors proliferated in response to human soluble EPO. These cells were analyzed to determine which STAT molecules were phosphorylated in response to EPO signaling. Briefly, control unmodified parental BAF-3 cells and EPOR/MU chimeric BAF-3 cells were rested from IL-3 containing growth medium, and restimulated with either IL-3 or EPO for 0, 15, 30 and 60 minutes. The cells were pelleted and resuspended in ice cold lysis buffer containing orthovanadate, to preserve phosphorylated tyrosines. Equal amounts of cell lysate were electrophoresed by SDS-PAGE and blotted onto nitrocellulose membranes for western analysis. Duplicate blots were stained for phosphorylated and nonphosphorylated forms of STAT 1, 3, 5, and 6 by using antibodies specific for each form of the STAT molecule. HELA cells, non-activated and activated with alpha-interferon were used as positive controls.

These results indicated that under these specific conditions, signaling through MU-1 results in the phosphorylation of STAT 5 at all time-points tested (T=0, T=15', T=30', T=60'). Treatment of controls or the chimeric BAF-3 cells with IL-3 resulted in phosphorylation of STAT 3, but not STAT 1 or 5.

### Example 4: Tissue Expression of Murine and Human MU-1

### Northern Analysis

Northern blots of polyA+ RNA from various tissues (Clonetech, Palo Alto, CA) were performed as recommended by the manufacturer. For the murine blots, a 262 nucleotide fragment corresponding to nucleotides 781-1043 of Fig. 1 and SEQ ID NO:9 was used for hybridization.

A single transcript of murine MU-1 was detected in adult murine spleen, lung, and heart tissues. The larger transcript observed in human tissues was not observed in mouse tissues.

Two transcripts of human MU-1 were detected in adult human lymphoid tissues, PBLs, thymus, spleen and lymph node, and in fetal lung.

### In Situ Hybridization

In situ hybridization studies were performed by Phylogency Inc. of Columbus, OH (according to the method of Lyons et..al. , 1990, J. Cell. Biol: 111:2427-2436.) Briefly, serial 5-7 micron paraffin sections were deparaffinized, fixed, digested with proteinase K, treated with tri-ethanolamine and dehydrated. cRNAs were prepared from linearized cDNA templates to generate antisense and sense probes. The cRNA transcripts were synthesized according to manufacturer's conditions (Ambion) and labeled with 35S-UTP. Sections were hybridized overnight, stringently washed and treated with RNAase A and dipped in nuclear track emulsion and exposed for 2-3 weeks. Control sections were hybridized with sense probes to indicate the background level of the procedure. The murine probe consisted of an 186bp fragment corresponding to nucleotides 860-1064 (SEQ ID NO:9). The human probe was a 231bp PCR product generated from human MU-1 DNA.

Murine MU-1 expression was observed in the lymph nodes of the adult small intestine at germinal centers and muscularis externa. Specialized lymph nodes and Peyers patches also exhibited murine MU-1 expression.

Human MU-1 expression was detected at germinal centers of the lymph nodules in the cortex. The medulla, which contains macrophages, was negative for human MU-1. In human spleen, human MU-1 expression was detected in the regions of white pulp but not red pulp.

### Example 5: Expression of Human MU-1 in cells and cell lines:

RNAse protection analysis was performed on resting and activated human T cells and the B cell lines, Raji and RPMI 8866, and the T cell line Jurkat. Human T cells were activated with anti-CD3 and anti-CD28. The cell lines were activated by Phorbol ester and ionomycin. MU-1 riboprobe-producing plasmid was constructed by inserting a 231bp PCR product (PCR was performed by using 5' primer CACAAAGCTTCAGTATGAGCTGCAGTACAGGAACCGGGGA (SEQ ID NO: 15) and 3' primer CACAGGATCCCTTTAACTCCTCTGACTGGGTCTGAAAGAT (SEQ ID NO:16)) into the BamH1 and HindIII sites of pGEM3zf(-) (Promega, Madison, WI) vector. To make the riboprobe, the riboprobe-producing plasmid was linearized with HindIII. The resulting DNA was phenol/chloroform extracted and precipitated with ethanol. T7 RNA polymerase was used to make the riboprobe according to the protocol suggested by the vendor (PharMingen, San Diego, CA). The RNAse protection assay was performed by using PharMingen's RiboQuant Multi-Probe Ribonuclease Protection Assay system. 2.0ug of total RNA were included in each RPA reaction, after RNAse digestion, the protected riboprobes were run on a QuickPoint rapid nucleic acid separation system (Novex, San Diego, CA). Gels were dried and exposed according to the suggestion of the vendor.

Human MU-1 RNA is upregulated in anti-CD3 +anti-CD28 stimulated human purified CD3+ cells when compared with unstimulated populations. MU-1 is also upregulated upon restimulation in Th and Th2-skewed T cell populations. The B cell lines, RPMI 8866 and Raji, constitutively express MU-1 while the Jurkat T cell line does not

### Example 6: Binding of Human MU-1 to Known Cytokines

Both human and murine Ig fusion proteins were constructed and immobilized on Biacore chips in an effort to identify the ligand for MU-1. A variety of cell culture conditioned media as well as a panel of known cytokines were evaluated for binding to MU-1. Some cytokines were also tested in combination with other receptor chains in the family to consider the possibility that MU-1 may require a second receptor chain for ligand binding. The following cytokines were tested and found to be negative for MU-1 binding: mIL-2, hIL-2, hIL-15, mIL-7, TSLP, TSLP+IL7, TSLP+IL7R, TSLP+IL7g, TSLP+ IL-2, TSLP + IL2 + IL2Rbeta, IL2Rbeta, IL2Rgamma, IL7R, IL2+2Rbeta, IL2+2Rgamma, IL15+IL2Rbeta, IL15+2Rgamma, IL7+2Rgamma, IL2+IL7R, IL15+IL7R, IL7+IL7R. Known receptors have been immobilized as well and tested for MUFc binding with negative results. IL-15 will bind to IL2Rb but not IL2Rg or MUFc.

## Claims

1. A method for identifying a compound capable of treating an immune disorder comprising assaying the ability of the compound to modulate MU-1 polypeptide interaction with a STAT molecule, thereby identifying a compound capable of treating an immune disorder, wherein the MU-1 polypeptide is a MU-1 protein family member having an amino acid sequence at least 65% identical to the amino acid sequence of SEQ ID NO:2 or 10.

2. The method of Claim 1, wherein the STAT molecule is STAT 3 or STAT 5.

3. A method of identifying a modulator of MU-1 activity comprising contacting a cell expressing a MU-1 protein according to SEQ ID NO:2 or 10, or a portion thereof capable of interaction with STAT molecules, with a test compound and determining the ability of said test compound to modulate interaction of said MU-1 protein, or said portion thereof, with a STAT molecule.

4. The method of claim 3, wherein said STAT molecule is STAT 3 or STAT 5.

5. The method of claim 3 or 4, wherein the MU-1 protein is according to SEQ ID NO:2 and is expressed in said cell as a fusion of the MCT-1 intracellular domain and the human EPO receptor extracellular domain.

## Patentansprüche

1. Verfahren zur Identifizierung einer Verbindung, die in der Lage ist, eine Immunerkrankung zu behandeln, umfassend das Prüfen der Fähigkeit der Verbindung, die Interaktion eines MU-1-Polypeptids mit einem STAT-Molekül zu modulieren, wobei **dadurch** eine Verbindung identifiziert wird, die in der Lage ist, eine Immunerkrankung zu behandeln, wobei es sich bei dem MU-1-Polypeptid um ein Mitglied der MU-1-Proteinfamilie mit einer Aminosäuresequenz handelt, die mindestens 65% identisch zur Aminosäuresequenz von SEQ ID Nr.:2 oder 10 ist.

2. Das Verfahren nach Anspruch 1, wobei es sich bei dem STAT-Molekül um STAT 3 oder STAT 5 handelt.

3. Verfahren zur Identifizierung eines Modulators der MU-1-Aktivität, umfassend das in Kontakt bringen einer Zelle, die ein MU-1-Protein entsprechend SEQ ID Nr.:2 oder 10 exprimiert, oder einen Teil davon, der in der Lage ist, mit STAT-Molekülen zu interagieren, mit einer Testverbindung und Bestimmen der Fähigkeit der genannten Testverbindung, die Interaktion des genannten MU-1-Proteins, oder des genannten Teils davon, mit einem STAT-Molekül zu modulieren.

4. Das Verfahren nach Anspruch 3, wobei es sich bei dem genannten STAT-Molekül um STAT 3 oder STAT 5 handelt.

5. Das Verfahren nach Anspruch 3 oder 4, wobei das MU-1-Protein eines entsprechend SEQ ID Nr.:2 ist und in der genannten Zelle als Fusion der intrazellulären Domäne von MU-1 und der extrazellulären Domäne des humanen EPO-Rezeptors exprimiert wird.

## Revendications

1. Procédé pour identifier un composé permettant de traiter un trouble immunitaire, comprenant l'analyse de l'aptitude du composé à moduler l'interaction du polypeptide MU-1 avec une molécule STAT, en identifiant ainsi un composé capable de traiter un trouble immunitaire, dans lequel le polypeptide MU-1 est un membre de la famille des protéines MU-1 ayant une séquence d'aminoacides identique à au moins 65 % à la séquence d'aminoacides de la SEQ ID N° 2 ou 10.

2. Procédé suivant la revendication 1, dans lequel la molécule STAT est la molécule STAT 3 ou STAT 5.

3. Procédé pour identifier un modulateur de l'activité de MU-1, comprenant la mise en contact d'une cellule exprimant une protéine MU-1 correspondant à la SEQ ID N° 2 ou 10, ou une partie de celle-ci capable d'interagir avec des molécules STAT, avec un composé d'essai, et la détermination de l'aptitude dudit composé d'essai à moduler l'interaction de ladite protéine MU-1, ou de ladite partie de celle-ci, avec une molécule STAT.

4. Procédé suivant la revendication 3, dans lequel ladite molécule STAT est la molécule STAT 3 ou STAT 5.

5. Procédé suivant la revendication 3 ou 4, dans lequel la protéine MU-1 correspond à la SEQ ID N° 2 et est exprimée dans ladite cellule sous forme d'une fusion du domaine intracellulaire de MU-1 et du domaine extracellulaire du récepteur d'EPO humain.


### SEQUENCE LISTING

<110> Genetics Institute, Inc., et al.
<120> MU-1, MEMBER OF THE CYTOKINE RECEPTOR FAMILY
<130> GFN-5230CPPC
<140>
   <141>
<150> 09/569,384
   <151> 2000-05-11
<160> 16
<170> PatentIn Ver. 2.0
<210> 1
   <211> 2665
   <212> DNA
   <213> Homo sapiens
<400> 1
   gtcgactgga ggcccagctg cccgtcatca gagtgacagg tcttatgaca gcctgattgg 60
   tgactcgggc tgggtgtgga ttctcacccc aggcctctgc ctgctttctc agaccctcat 120
   ctgtcacccc cacgctgaac ccagctgcca cccccagaag cccatcagac tgcccccagc 180
   acacggaatg gatttctgag aaagaagccg aaacagaagg cccgtgggag tcagcatgcc 240
   gcgtggctgg gccgccccct tgctcctgct gctgctccag ggaggctggg gctgccccga 300
   cctcgtctgc tacaccgatt acctccagac ggtcatctgc atcctggaaa tgtggaacct 360
   ccaccccagc acgctcaccc ttacctggca agaccagtat gaagagctga aggacgaggc 420
   cacctcctgc agcctccaca ggtcggccca caatgccacg catgccacct acacctgcca 480
   catggatgta ttccacttca tggccgacga cattttcagt gtcaacatca cagaccagtc 540
   tggcaactac tcccaggagt gtggcagctt tctcctggct gagagcatca agccggctcc 600
   ccctttcaac gtgactgtga ccttctcagg acagtataat atctcctggc gctcagatta 660
   cgaagaccct gccttctaca tgctgaaggg caagcttcag tatgagctgc agtacaggaa 720
   ccggggagac ccctgggctg tgagtccgag gagaaagctg atctcagtgg actcaagaag 780
   tgtctccctc ctccccctgg agttccgcaa agactcgagc tatgagctgc aggtgcgggc 840
   agggcccatg cctggctcct cctaccaggg gacctggagt gaatggagtg acccggtcat 900
   ctttcagacc cagtcagagg agttaaagga aggctggaac cctcacctgc tgcttctcct 960
   cctgcttgtc atagtcttca ttcctgcctt ctggagcctg aagacccatc cattgtggag 1020
   gctatggaag aagatatggg ccgtccccag ccctgagcgg ttcttcatgc ccctgtacaa 1080
   gggctgcagc ggagacttca agaaatgggt gggtgcaccc ttcactggct ccagcctgga 1140
   gctgggaccc tggagcccag aggtgccctc caccctggag gtgtacagct gccacccacc 1200
   acggagcccg gccaagaggc tgcagctcac ggagctacaa gaaccagcag agctggtgga 1260
   gtctgacggt gtgcccaagc ccagcttctg gccgacagcc cagaactcgg ggggctcagc 1320
   ttacagtgag gagagggatc ggccatacgg cctggtgtcc attgacacag tgactgtgct 1380
   agatgcagag gggccatgca cctggccctg cagctgtgag gatgacggct acccagccct 1440
   ggacctggat gctggcctgg agcccagccc aggcctagag gacccactct tggatgcagg 1500
   gaccacagtc ctgtcctgtg gctgtgtctc agctggcagc cctgggctag gagggcccct 1560
   gggaagcctc ctggacagac taaagccacc ccttgcagat ggggaggact gggctggggg 1620
   actgccctgg ggtggccggt cacctggagg ggtctcagag agtgaggcgg gctcacccct 1680
   ggccggcctg gatatggaca cgtttgacag tggctttgtg ggctctgact gcagcagccc 1740
   tgtggagtgt gacttcacca gccccgggga cgaaggaccc ccccggagct acctccgcca 1800
   gtgggtggtc attcctccgc cactttcgag ccctggaccc caggccagct aatgaggctg 1860
   actggatgtc cagagctggc caggccactg ggccctgagc cagagacaag gtcacctggg 1920
   ctgtgatgtg aagacacctg cagcctttgg tctcctggat gggcctttga gcctgatgtt 1980
   tacagtgtct gtgtgtgtgt gtgcatatgt gtgtgtgtgc atatgcatgt gtgtgtgtgt 2040
   gtgtgtctta ggtgcgcagt ggcatgtcca cgtgtgtgtg tgattgcacg tgcctgtggg 2100
   cctgggataa tgcccatggt actccatgca ttcacctgcc ctgtgcatgt ctggactcac 2160
   ggagctcacc catgtgcaca agtgtgcaca gtaaacgtgt ttgtggtcaa cagatgacaa 2220
   cagccgtcct ccctcctagg gtcttgtgtt gcaagttggt ccacagcatc tccggggctt 2280
   tgtgggatca gggcattgcc tgtgactgag gcggagccca gccctccagc gtctgcctcc 2340
   aggagctgca agaagtccat attgttcctt atcacctgcc aacaggaagc gaaaggggat 2400
   ggagtgagcc catggtgacc tcgggaatgg caattttttg ggcggcccct ggacgaaggt 2460
   ctgaatcccg actctgatac cttctggctg tgctacctga gccaagtcgc ctcccctctc 2520
   tgggctagag tttccttatc cagacagtgg ggaaggcatg acacacctgg gggaaattgg 2580
   cgatgtcacc cgtgtacggt acgcagccca gagcagaccc tcaataaacg tcagcttcct 2640
   tcaaaaaaaa aaaaaaaaat ctaga 2665
<210> 2
   <211> 538
   <212> PRT
   <213> Homo sapiens
<400> 2
   Met Pro Arg Gly Trp Ala Ala Pro Leu Leu Leu Leu Leu Leu Gln Gly
    1               5                  10                  15
   Gly Trp Gly Cys Pro Asp Leu Val Cys Tyr Thr Asp Tyr Leu Gln Thr
               20                  25                  30
   Val Ile Cys Ile Leu Glu Met Trp Asn Leu His Pro Ser Thr Leu Thr
           35                  40                  45
   Leu Thr Trp Gln Asp Gln Tyr Glu Glu Leu Lys Asp Glu Ala Thr Ser
       50                  55                  60
   Cys Ser Leu His Arg Ser Ala His Asn Ala Thr His Ala Thr Tyr Thr
    65                  70                  75                  80
   Cys His Met Asp Val Phe His Phe Met Ala Asp Asp Ile Phe Ser Val
                    85                  90                  95
   Asn Ile Thr Asp Gln Ser Gly Asn Tyr Ser Gln Glu Cys Gly Ser Phe
               100                 105                 110
   Leu Leu Ala Glu Ser Ile Lys Pro Ala Pro Pro Phe Asn Val Thr Val
           115                 120                 125
   Thr Phe Ser Gly Gln Tyr Asn Ile Ser Trp Arg Ser Asp Tyr Glu Asp
       130                 135                 140
   Pro Ala Phe Tyr Met Leu Lys Gly Lys Leu Gln Tyr Glu Leu Gln Tyr
   145                 150                 155                 160
   Arg Asn Arg Gly Asp Pro Trp Ala Val Ser Pro Arg Arg Lys Leu Ile
                   165                 170                 175
   Ser Val Asp Ser Arg Ser Val Ser Leu Leu Pro Leu Glu Phe Arg Lys
               180                 185                 190
   Asp Ser Ser Tyr Glu Leu Gln Val Arg Ala Gly Pro Met Pro Gly Ser
           195                 200                 205
   Ser Tyr Gln Gly Thr Trp Ser Glu Trp Ser Asp Pro Val Ile Phe Gln
       210                 215                 220
   Thr Gln Ser Glu Glu Leu Lys Glu Gly Trp Asn Pro His Leu Leu Leu
   225                 230                 235                 240
   Leu Leu Leu Leu Val Ile Val Phe Ile Pro Ala Phe Trp Ser Leu Lys
                   245                 250                 255
   Thr His Pro Leu Trp Arg Leu Trp Lys Lys Ile Trp Ala Val Pro Ser
               260                 265                 270
   Pro Glu Arg Phe Phe Met Pro Leu Tyr Lys Gly Cys Ser Gly Asp Phe
           275                 280                 285
   Lys Lys Trp Val Gly Ala Pro Phe Thr Gly Ser Ser Leu Glu Leu Gly
       290                 295                 300
   Pro Trp Ser Pro Glu Val Pro Ser Thr Leu Glu Val Tyr Ser Cys His
   305                 310                 315                 320
   Pro Pro Arg Ser Pro Ala Lys Arg Leu Gln Leu Thr Glu Leu Gln Glu
                   325                 330                 335
   Pro Ala Glu Leu Val Glu Ser Asp Gly Val Pro Lys Pro Ser Phe Trp
               340                 345                 350
   Pro Thr Ala Gln Asn Ser Gly Gly Ser Ala Tyr Ser Glu Glu Arg Asp
           355                 360                 365
   Arg Pro Tyr Gly Leu Val Ser Ile Asp Thr Val Thr Val Leu Asp Ala
       370                 375                 380
   Glu Gly Pro Cys Thr Trp Pro Cys Ser Cys Glu Asp Asp Gly Tyr Pro
   385                 390                 395                 400
   Ala Leu Asp Leu Asp Ala Gly Leu Glu Pro Ser Pro Gly Leu Glu Asp
                   405                 410                 415
   Pro Leu Leu Asp Ala Gly Thr Thr Val Leu Ser Cys Gly Cys Val Ser
               420                 425                 430
   Ala Gly Ser Pro Gly Leu Gly Gly Pro Leu Gly Ser Leu Leu Asp Arg
           435                 440                 445
   Leu Lys Pro Pro Leu Ala Asp Gly Glu Asp Trp Ala Gly Gly Leu Pro
       450                 455                 460
   Trp Gly Gly Arg Ser Pro Gly Gly Val Ser Glu Ser Glu Ala Gly Ser
   465                 470                 475                 480
   Pro Leu Ala Gly Leu Asp Met Asp Thr Phe Asp Ser Gly Phe Val Gly
                   485                 490                 495
   Ser Asp Cys Ser Ser Pro Val Glu Cys Asp Phe Thr Ser Pro Gly Asp
               500                 505                 510
   Glu Gly Pro Pro Arg Ser Tyr Leu Arg Gln Trp Val Val Ile Pro Pro
           515                 520                 525
   Pro Leu Ser Ser Pro Gly Pro Gln Ala Ser
       530                 535
<210> 3
   <211> 70
   <212> PRT
   <213> Homo sapiens
<400> 3
   Leu Met Thr Asn Ala Phe Ile Ser Ile Ile Asp Asp Leu Ser Lys Tyr
   1               5                   10                  15
   Asp Val Gln Val Arg Ala Ala Val Ser Ser Met Cys Arg Glu Ala Gly
               20                  25                  30
   Leu Trp Ser Glu Trp Ser Gln Pro Ile Tyr Val Gly Asn Asp Glu His
           35                  40                  45
   Lys Pro Leu Arg Glu Trp Phe Val Ile Val Ile Met Ala Thr Ile Cys
       50                  55                  60
   Phe Ile Leu Leu Ile Leu
   65                  70
<210> 4
   <211> 25
   <212> DNA
   <213> Homo sapiens
<400> 4
   gagtccgagg agaaagctga tctca 25
<210> 5
   <211> 24
   <212> DNA
   <213> Homo sapiens
<400> 5
   gaaagatgac cgggtcactc catt 24
<210> 6
   <211> 29
   <212> DNA
   <213> Homo sapiens
<400> 6
   actcgagcta tgagctgcag gtgcgggca 29
<210> 7
   <211> 29
   <212> DNA
   <213> Homo sapiens
<400> 7
   actcgagcta tgagctgcag gtgcgggca 29
<210> 8
   <211> 5
   <212> PRT
   <213> Homo sapiens
<220>
   <223> Xaa at position 3 may be any amino acid
<400> 8
   Trp Ser Xaa Trp Ser
   1               5
<210> 9
   <211> 2628
   <212> DNA
   <213> Mus musculus
<400> 9
   gtcgacgcgg cggtaccagc tgtctgccca cttctcctgt ggtgtgcctc acggtcactt 60
   gcttgtctga ccgcaagtct gcccatccct ggggcagcca actggcctca gcccgtgccc 120
   caggcgtgcc ctgtctctgt ctggctgccc cagccctact gtcttcctct gtgtaggctc 180
   tgcccagatg cccggctggt cctcagcctc aggactatct cagcagtgac tcccctgatt 240
   ctggacttgc acctgactga actcctgccc acctcaaacc ttcacctccc accaccacca 300
   ctccgagtcc cgctgtgact cccacgccca ggagaccacc caagtgcccc agcctaaaga 360
   atggctttct gagaaagacc ctgaaggagt aggtctggga cacagcatgc cccggggccc 420
   agtggctgcc ttactcctgc tgattctcca tggagcttgg agctgcctgg acctcacttg 480
   ctacactgac tacctctgga ccatcacctg tgtcctggag acacggagcc ccaaccccag 540
   catactcagt ctcacctggc aagatgaata tgaggaactt caggaccaag agaccttctg 600
   cagcctacac aggtctggcc acaacaccac acatatatgg tacacgtgcc atatgcgctt 660
   gtctcaattc ctgtccgatg aagttttcat tgtcaatgtg acggaccagt ctggcaacaa 720
   ctcccaagag tgtggcagct ttgtcctggc tgagagcatc aaaccagctc cccccttgaa 780
   cgtgactgtg gccttctcag gacgctatga tatctcctgg gactcagctt atgacgaacc 840
   ctccaactac gtgctgaggg gcaagctaca atatgagctg cagtatcgga acctcagaga 900
   cccctatgct gtgaggccgg tgaccaagct gatctcagtg gactcaagaa acgtctctct 960
   tctccctgaa gagttccaca aagattctag ctaccagctg caggtgcggg cagcgcctca 1020
   gccaggcact tcattcaggg ggacctggag tgagtggagt gaccccgtca tctttcagac 1080
   ccaggctggg gagcccgagg caggctggga ccctcacatg ctgctgctcc tggctgtctt 1140
   gatcattgtc ctggttttca tgggtctgaa gatccacctg ccttggaggc tatggaaaaa 1200
   gatatgggca ccagtgccca cccctgagag tttcttccag cccctgtaca gggagcacag 1260
   cgggaacttc aagaaatggg ttaatacccc tttcacggcc tccagcatag agttggtgcc 1320
   acagagttcc acaacaacat cagccttaca tctgtcattg tatccagcca aggagaagaa 1380
   gttcccgggg ctgccgggtc tggaagagca actggagtgt gatggaatgt ctgagcctgg 1440
   tcactggtgc ataatcccct tggcagctgg ccaagcggtc tcagcctaca gtgaggagag 1500
   agaccggcca tatggtctgg tgtccattga cacagtgact gtgggagatg cagagggcct 1560
   gtgtgtctgg ccctgtagct gtgaggatga tggctatcca gccatgaacc tggatgctgg 1620
   ccgagagtct ggccctaatt cagaggatct gctcttggtc acagaccctg cttttctgtc 1680
   ttgcggctgt gtctcaggta gtggtctcag gcttggaggc tccccaggca gcctactgga 1740
   caggttgagg ctgtcatttg caaaggaagg ggactggaca gcagacccaa cctggagaac 1800
   tgggtcccca ggagggggct ctgagagtga agcaggttcc ccccctggtc tggacatgga 1860
   cacatttgac agtggctttg caggttcaga ctgtggcagc cccgtggaga ctgatgaagg 1920
   accccctcga agctatctcc gccagtgggt ggtcaggacc cctccacctg tggacagtgg 1980
   agcccagagc agctagcata taataaccag ctatagtgag aagaggcctc tgagcctggc 2040
   atttacagtg tgaacatgta ggggtgtgtg tgtgtgtgtg tgtgtgtgtg tgtgtgtgtg 2100
   tgtgtgtgtg tgtgtgtgtg tgtcttgggt tgtgtgttag cacatccatg ttgggatttg 2160
   gtctgttgct atgtattgta atgctaaatt ctctacccaa agttctaggc ctacgagtga 2220
   attctcatgt ttacaaactt gctgtgtaaa ccttgttcct taatttaata ccattggtta 2280
   aataaaattg gctgcaacca attactggag ggattagagg tagggggctt ttgagttacc 2340
   tgtttggaga tggagaagga gagaggagag accaagagga gaaggaggaa ggagaggaga 2400
   ggagaggaga ggagaggaga ggagaggaga ggagaggaga ggagaggaga ggctgccgtg 2460
   aggggagagg gaccatgagc ctgtggccag gagaaacagc aagtatctgg ggtacactgg 2520
   tgaggaggtg gccaggccag cagttagaag agtagattag gggtgacctc cagtatttgt 2580
   caaagccaat taaaataaca aaaaaaaaaa aaaagcggcc gctctaga 2628
<210> 10
   <211> 529
   <212> PRT
   <213> Mus musculus
<400> 10
   Met Pro Arg Gly Pro Val Ala Ala Leu Leu Leu Leu Ile Leu His Gly
    1               5                  10                  15
   Ala Trp Ser Cys Leu Asp Leu Thr Cys Tyr Thr Asp Tyr Leu Trp Thr
               20                  25                  30
   Ile Thr Cys Val Leu Glu Thr Arg Ser Pro Asn Pro Ser Ile Leu Ser
           35                  40                  45
   Leu Thr Trp Gln Asp Glu Tyr Glu Glu Leu Gln Asp Gln Glu Thr Phe
       50                  55                  60
   Cys Ser Leu His Arg Ser Gly His Asn Thr Thr His Ile Trp Tyr Thr
   65                  70                  75                  80
   Cys His Met Arg Leu Ser Gln Phe Leu Ser Asp Glu Val Phe Ile Val
                   85                  90                  95
   Asn Val Thr Asp Gln Ser Gly Asn Asn Ser Gln Glu Cys Gly Ser Phe
               100                 105                 110
   Val Leu Ala Glu Ser Ile Lys Pro Ala Pro Pro Leu Asn Val Thr Val
           115                 120                 125
   Ala Phe Ser Gly Arg Tyr Asp Ile Ser Trp Asp Ser Ala Tyr Asp Glu
       130                 135                 140
   Pro Ser Asn Tyr Val Leu Arg Gly Lys Leu Gln Tyr Glu Leu Gln Tyr
   145                 150                 155                 160
   Arg Asn Leu Arg Asp Pro Tyr Ala Val Arg Pro Val Thr Lys Leu Ile
                   165                 170                 175
   Ser Val Asp Ser Arg Asn Val Ser Leu Leu Pro Glu Glu Phe His Lys
               180                 185                 190
   Asp Ser Ser Tyr Gln Leu Gln Val Arg Ala Ala Pro Gln Pro Gly Thr
           195                 200                 205
   Ser Phe Arg Gly Thr Trp Ser Glu Trp Ser Asp Pro Val Ile Phe Gln
       210                 215                 220
   Thr Gln Ala Gly Glu Pro Glu Ala Gly Trp Asp Pro His Met Leu Leu
   225                 230                 235                 240
   Leu Leu Ala Val Leu Ile Ile Val Leu Val Phe Met Gly Leu Lys Ile
                   245                 250                 255
   His Leu Pro Trp Arg Leu Trp Lys Lys Ile Trp Ala Pro Val Pro Thr
               260                 265                 270
   Pro Glu Ser Phe Phe Gln Pro Leu Tyr Arg Glu His Ser Gly Asn Phe
           275                 280                 285
   Lys Lys Trp Val Asn Thr Pro Phe Thr Ala Ser Ser Ile Glu Leu Val
       290                 295                 300
   Pro Gln Ser Ser Thr Thr Thr Ser Ala Leu His Leu Ser Leu Tyr Pro
   305                 310                 315                 320
   Ala Lys Glu Lys Lys Phe Pro Gly Leu Pro Gly Leu Glu Glu Gln Leu
                   325                 330                 335
   Glu Cys Asp Gly Met Ser Glu Pro Gly His Trp Cys Ile Ile Pro Leu
               340                 345                 350
   Ala Ala Gly Gln Ala Val Ser Ala Tyr Ser Glu Glu Arg Asp Arg Pro
           355                 360                 365
   Tyr Gly Leu Val Ser Ile Asp Thr Val Thr Val Gly Asp Ala Glu Gly
       370                 375                 380
   Leu Cys Val Trp Pro Cys Ser Cys Glu Asp Asp Gly Tyr Pro Ala Met
   385                 390                 395                 400
   Asn Leu Asp Ala Gly Arg Glu Ser Gly Pro Asn Ser Glu Asp Leu Leu
                   405                 410                 415
   Leu Val Thr Asp Pro Ala Phe Leu Ser Cys Gly Cys Val Ser Gly Ser
               420                 425                 430
   Gly Leu Arg Leu Gly Gly Ser Pro Gly Ser Leu Leu Asp Arg Leu Arg
           435                 440                 445
   Leu Ser Phe Ala Lys Glu Gly Asp Trp Thr Ala Asp Pro Thr Trp Arg
       450                 455                 460
   Thr Gly Ser Pro Gly Gly Gly Ser Glu Ser Glu Ala Gly Ser Pro Pro
   465                 470                 475                 480
   Gly Leu Asp Met Asp Thr Phe Asp Ser Gly Phe Ala Gly Ser Asp Cys
                   485                 490                 495
   Gly Ser Pro Val Glu Thr Asp Glu Gly Pro Pro Arg Ser Tyr Leu Arg
               500                 505                 510
   Gln Trp Val Val Arg Thr Pro Pro Pro Val Asp Ser Gly Ala Gln Ser
           515                 520                 525
   Ser
<210> 11
   <211> 20
   <212> DNA
   <213> Homo sapiens
<400> 11
   agcatcaagc cggctccccc 20
<210> 12
   <211> 20
   <212> DNA
   <213> Homo sapiens
<400> 12
   ctccattcac tccaggtccc 20
<210> 13
   <211> 20
   <212> DNA
   <213> Mus musculus
<400> 13
   ttgaacgtga ctgtggcctt 20
<210> 14
   <211> 20
   <212> DNA
   <213> Mus musculus
<400> 14
   tgaatgaagt gcctggctga 20
<210> 15
   <211> 40
   <212> DNA
   <213> Homo sapiens
<400> 15
   cacaaagctt cagtatgagc tgcagtacag gaaccgggga 40
<210> 16
   <211> 40
   <212> DNA
   <213> Homo sapiens
<400> 16
   cacaggatcc ctttaactcc tctgactggg tctgaaagat 40

### SEQUENCE LISTING

<110> Genetics Institute, Inc., et al.
<120> MU-1, MEMBER OF THE CYTOKINE RECEPTOR FAMILY
<130> GFN-5230CPPC
<140>
   <141>
<150> 09/569,384
   <151> 2000-05-11
<160> 16
<170> PatentIn Ver. 2.0
<210> 1
   <211> 2665
   <212> DNA
   <213> Homo sapiens
<400> 1
   gtcgactgga ggcccagctg cccgtcatca gagtgacagg tcttatgaca gcctgattgg 60
   tgactcgggc tgggtgtgga ttctcacccc aggcctctgc ctgctttctc agaccctcat 120
   ctgtcacccc cacgctgaac ccagctgcca cccccagaag cccatcagac tgcccccagc 180
   acacggaatg gatttctgag aaagaagccg aaacagaagg cccgtgggag tcagcatgcc 240
   gcgtggctgg gccgccccct tgctcctgct gctgctccag ggaggctggg gctgccccga 300
   cctcgtctgc tacaccgatt acctccagac ggtcatctgc atcctggaaa tgtggaacct 360
   ccaccccagc acgctcaccc ttacctggca agaccagtat gaagagctga aggacgaggc 420
   cacctcctgc agcctccaca ggtcggccca caatgccacg catgccacct acacctgcca 480
   catggatgta ttccacttca tggccgacga cattttcagt gtcaacatca cagaccagtc 540
   tggcaactac tcccaggagt gtggcagctt tctcctggct gagagcatca agccggctcc 600
   ccctttcaac gtgactgtga ccttctcagg acagtataat atctcctggc gctcagatta 660
   cgaagaccct gccttctaca tgctgaaggg caagcttcag tatgagctgc agtacaggaa 720
   ccggggagac ccctgggctg tgagtccgag gagaaagctg atctcagtgg actcaagaag 780
   tgtctccctc ctccccctgg agttccgcaa agactcgagc tatgagctgc aggtgcgggc 840
   agggcccatg cctggctcct cctaccaggg gacctggagt gaatggagtg acccggtcat 900
   ctttcagacc cagtcagagg agttaaagga aggctggaac cctcacctgc tgcttctcct 960
   cctgcttgtc atagtcttca ttcctgcctt ctggagcctg aagacccatc cattgtggag 1020
   gctatggaag aagatatggg ccgtccccag ccctgagcgg ttcttcatgc ccctgtacaa 1080
   gggctgcagc ggagacttca agaaatgggt gggtgcaccc ttcactggct ccagcctgga 1140
   gctgggaccc tggagcccag aggtgccctc caccctggag gtgtacagct gccacccacc 1200
   acggagcccg gccaagaggc tgcagctcac ggagctacaa gaaccagcag agctggtgga 1260
   gtctgacggt gtgcccaagc ccagcttctg gccgacagcc cagaactcgg ggggctcagc 1320
   ttacagtgag gagagggatc ggccatacgg cctggtgtcc attgacacag tgactgtgct 1380
   agatgcagag gggccatgca cctggccctg cagctgtgag gatgacggct acccagccct 1440
   ggacctggat gctggcctgg agcccagccc aggcctagag gacccactct tggatgcagg 1500
   gaccacagtc ctgtcctgtg gctgtgtctc agctggcagc cctgggctag gagggcccct 1560
   gggaagcctc ctggacagac taaagccacc ccttgcagat ggggaggact gggctggggg 1620
   actgccctgg ggtggccggt cacctggagg ggtctcagag agtgaggcgg gctcacccct 1680
   ggccggcctg gatatggaca cgtttgacag tggctttgtg ggctctgact gcagcagccc 1740
   tgtggagtgt gacttcacca gccccgggga cgaaggaccc ccccggagct acctccgcca 1800
   gtgggtggtc attcctccgc cactttcgag ccctggaccc caggccagct aatgaggctg 1860
   actggatgtc cagagctggc caggccactg ggccctgagc cagagacaag gtcacctggg 1920
   ctgtgatgtg aagacacctg cagcctttgg tctcctggat gggcctttga gcctgatgtt 1980
   tacagtgtct gtgtgtgtgt gtgcatatgt gtgtgtgtgc atatgcatgt gtgtgtgtgt 2040
   gtgtgtctta ggtgcgcagt ggcatgtcca cgtgtgtgtg tgattgcacg tgcctgtggg 2100
   cctgggataa tgcccatggt actccatgca ttcacctgcc ctgtgcatgt ctggactcac 2160
   ggagctcacc catgtgcaca agtgtgcaca gtaaacgtgt ttgtggtcaa cagatgacaa 2220
   cagccgtcct ccctcctagg gtcttgtgtt gcaagttggt ccacagcatc tccggggctt 2280
   tgtgggatca gggcattgcc tgtgactgag gcggagccca gccctccagc gtctgcctcc 2340
   aggagctgca agaagtccat attgttcctt atcacctgcc aacaggaagc gaaaggggat 2400
   ggagtgagcc catggtgacc tcgggaatgg caattttttg ggcggcccct ggacgaaggt 2460
   ctgaatcccg actctgatac cttctggctg tgctacctga gccaagtcgc ctcccctctc 2520
   tgggctagag tttccttatc cagacagtgg ggaaggcatg acacacctgg gggaaattgg 2580
   cgatgtcacc cgtgtacggt acgcagccca gagcagaccc tcaataaacg tcagcttcct 2640
   tcaaaaaaaa aaaaaaaaat ctaga                                      2665
<210> 2
   <211> 538
   <212> PRT
   <213> Homo sapiens
<400> 2
   Met Pro Arg Gly Trp Ala Ala Pro Leu Leu Leu Leu Leu Leu Gln Gly
    1               5                  10                  15
   Gly Trp Gly Cys Pro Asp Leu Val Cys Tyr Thr Asp Tyr Leu Gln Thr
               20                  25                  30
   Val Ile Cys Ile Leu Glu Met Trp Asn Leu His Pro Ser Thr Leu Thr
           35                  40                  45
   Leu Thr Trp Gln Asp Gln Tyr Glu Glu Leu Lys Asp Glu Ala Thr Ser
       50                  55                  60
   Cys Ser Leu His Arg Ser Ala His Asn Ala Thr His Ala Thr Tyr Thr
   65                  70                  75                  80
   Cys His Met Asp Val Phe His Phe Met Ala Asp Asp Ile Phe Ser Val
                   85                  90                  95
   Asn Ile Thr Asp Gln Ser Gly Asn Tyr Ser Gln Glu Cys Gly Ser Phe
               100                 105                 110
   Leu Leu Ala Glu Ser Ile Lys Pro Ala Pro Pro Phe Asn Val Thr Val
           115                 120                 125
   Thr Phe Ser Gly Gln Tyr Asn Ile Ser Trp Arg Ser Asp Tyr Glu Asp
       130                 135                 140
   Pro Ala Phe Tyr Met Leu Lys Gly Lys Leu Gln Tyr Glu Leu Gln Tyr
   145                 150                 155                 160
   Arg Asn Arg Gly Asp Pro Trp Ala Val Ser Pro Arg Arg Lys Leu Ile
                   165                 170                 175
   Ser Val Asp Ser Arg Ser Val Ser Leu Leu Pro Leu Glu Phe Arg Lys
               180                 185                 190
   Asp Ser Ser Tyr Glu Leu Gln Val Arg Ala Gly Pro Met Pro Gly Ser
           195                 200                 205
   Ser Tyr Gln Gly Thr Trp Ser Glu Trp Ser Asp Pro Val Ile Phe Gln
       210                 215                 220
   Thr Gln Ser Glu Glu Leu Lys Glu Gly Trp Asn Pro His Leu Leu Leu
   225                 230                 235                 240
   Leu Leu Leu Leu Val Ile Val Phe Ile Pro Ala Phe Trp Ser Leu Lys
                   245                     250                     255
   Thr His Pro Leu Trp Arg Leu Trp Lys Lys Ile Trp Ala Val Pro Ser
               260                     265                     270
   Pro Glu Arg Phe Phe Met Pro Leu Tyr Lys Gly Cys Ser Gly Asp Phe
           275                     280                     285
   Lys Lys Trp Val Gly Ala Pro Phe Thr Gly Ser Ser Leu Glu Leu Gly
       290                     295                     300
   Pro Trp Ser Pro Glu Val Pro Ser Thr Leu Glu Val Tyr Ser Cys His
   305                     310                     315                     320
   Pro Pro Arg Ser Pro Ala Lys Arg Leu Gln Leu Thr Glu Leu Gln Glu
                   325                     330                     335
   Pro Ala Glu Leu Val Glu Ser Asp Gly Val Pro Lys Pro Ser Phe Trp
               340                     345                     350
   Pro Thr Ala Gln Asn Ser Gly Gly Ser Ala Tyr Ser Glu Glu Arg Asp
           355                     360                     365
   Arg Pro Tyr Gly Leu Val Ser Ile Asp Thr Val Thr Val Leu Asp Ala
       370                     375                     380
   Glu Gly Pro Cys Thr Trp Pro Cys Ser Cys Glu Asp Asp Gly Tyr Pro
   385                     390                     395                     400
   Ala Leu Asp Leu Asp Ala Gly Leu Glu Pro Ser Pro Gly Leu Glu Asp
                   405                     410                     415
   Pro Leu Leu Asp Ala Gly Thr Thr Val Leu Ser Cys Gly Cys Val Ser
               420                     425                     430
   Ala Gly Ser Pro Gly Leu Gly Gly Pro Leu Gly Ser Leu Leu Asp Arg
           435                     440                     445
   Leu Lys Pro Pro Leu Ala Asp Gly Glu Asp Trp Ala Gly Gly Leu Pro
       450                     455                     460
   Trp Gly Gly Arg Ser Pro Gly Gly Val Ser Glu Ser Glu Ala Gly Ser
   465                     470                     475                     480
   Pro Leu Ala Gly Leu Asp Met Asp Thr Phe Asp Ser Gly Phe Val Gly
                   485                     490                     495
   Ser Asp Cys Ser Ser Pro Val Glu Cys Asp Phe Thr Ser Pro Gly Asp
               500                     505                     510
   Glu Gly Pro Pro Arg Ser Tyr Leu Arg Gln Trp Val Val Ile Pro Pro
           515                     520                     525
   Pro Leu Ser Ser Pro Gly Pro Gln Ala Ser
       530                     535
<210> 3
   <211> 70
   <212> PRT
   <213> Homo sapiens
<400> 3
   Leu Met Thr Asn Ala Phe Ile Ser Ile Ile Asp Asp Leu Ser Lys Tyr
   1 5 10 15
   Asp Val Gln Val Arg Ala Ala Val Ser Ser Met Cys Arg Glu Ala Gly
   20 25 30
   Leu Trp Ser Glu Trp Ser Gln Pro Ile Tyr Val Gly Asn Asp Glu His
   35 40 45
   Lys Pro Leu Arg Glu Trp Phe Val Ile Val Ile Met Ala Thr Ile Cys
   50 55 60
   Phe Ile Leu Leu Ile Leu
   65 70
<210> 4
   <211> 25
   <212> DNA
   <213> Homo sapiens
<400> 4
   gagtccgagg agaaagctga tctca 25
<210> 5
   <211> 24
   <212> DNA
   <213> Homo sapiens
<400> 5
   gaaagatgac cgggtcactc catt 24
<210> 6
   <211> 29
   <212> DNA
   <213> Homo sapiens
<400> 6
   actcgagcta tgagctgcag gtgcgggca 29
<210> 7
   <211> 29
   <212> DNA
   <213> Homo sapiens
<400> 7
   actcgagcta tgagctgcag gtgcgggca 29
<210> 8
   <211> 5
   <212> PRT
   <213> Homo sapiens
<220>
   <223> Xaa at position 3 may be any amino acid
<400> 8
   Trp Ser Xaa Trp Ser
   1 5
<210> 9
   <211> 2628
   <212> DNA
   <213> Mus musculus
<400> 9
<210> 10
   <211> 529
   <212> PRT
   <213> Mus musculus
<400> 10
   Met Pro Arg Gly Pro Val Ala Ala Leu Leu Leu Leu Ile Leu His Gly
   1               5                   10                  15
   Ala Trp Ser Cys Leu Asp Leu Thr Cys Tyr Thr Asp Tyr Leu Trp Thr
               20                  25                  30
   Ile Thr Cys Val Leu Glu Thr Arg Ser Pro Asn Pro Ser Ile Leu Ser
           35                  40                  45
   Leu Thr Trp Gln Asp Glu Tyr Glu Glu Leu Gln Asp Gln Glu Thr Phe
       50                  55                  60
   Cys Ser Leu His Arg Ser Gly His Asn Thr Thr His Ile Trp Tyr Thr
   65                  70                  75                  80
   Cys His Met Arg Leu Ser Gln Phe Leu Ser Asp Glu Val Phe Ile Val
                   85                  90                  95
   Asn Val Thr Asp Gln Ser Gly Asn Asn Ser Gln Glu Cys Gly Ser Phe
               100                 105                 110
   Val Leu Ala Glu Ser Ile Lys Pro Ala Pro Pro Leu Asn Val Thr Val
           115                 120                 125
   Ala Phe Ser Gly Arg Tyr Asp Ile Ser Trp Asp Ser Ala Tyr Asp Glu
       130                 135                 140
   Pro Ser Asn Tyr Val Leu Arg Gly Lys Leu Gln Tyr Glu Leu Gln Tyr
   145                 150                 155                 160
   Arg Asn Leu Arg Asp Pro Tyr Ala Val Arg Pro Val Thr Lys Leu Ile
                   165                 170                 175
   Ser Val Asp Ser Arg Asn Val Ser Leu Leu Pro Glu Glu Phe His Lys
               180                 185                 190
   Asp Ser Ser Tyr Gln Leu Gln Val Arg Ala Ala Pro Gln Pro Gly Thr
           195                 200                 205
   Ser Phe Arg Gly Thr Trp Ser Glu Trp Ser Asp Pro Val Ile Phe Gln
       210                 215                 220
   Thr Gln Ala Gly Glu Pro Glu Ala Gly Trp Asp Pro His Met Leu Leu
   225                 230                 235                 240
   Leu Leu Ala Val Leu Ile Ile Val Leu Val Phe Met Gly Leu Lys Ile
                   245                 250                 255
   His Leu Pro Trp Arg Leu Trp Lys Lys Ile Trp Ala Pro Val Pro Thr
               260                 265                 270
   Pro Glu Ser Phe Phe Gln Pro Leu Tyr Arg Glu His Ser Gly Asn Phe
           275                 280                 285
   Lys Lys Trp Val Asn Thr Pro Phe Thr Ala Ser Ser Ile Glu Leu Val
       290                 295                 300
   Pro Gln Ser Ser Thr Thr Thr Ser Ala Leu His Leu Ser Leu Tyr Pro
   305                 310                 315                 320
   Ala Lys Glu Lys Lys Phe Pro Gly Leu Pro Gly Leu Glu Glu Gln Leu
                   325                 330                 335
   Glu Cys Asp Gly Met Ser Glu Pro Gly His Trp Cys Ile Ile Pro Leu
               340                 345                 350
   Ala Ala Gly Gln Ala Val Ser Ala Tyr Ser Glu Glu Arg Asp Arg Pro
           355                 360                 365
   Tyr Gly Leu Val Ser Ile Asp Thr Val Thr Val Gly Asp Ala Glu Gly
       370                 375                 380
   Leu Cys Val Trp Pro Cys Ser Cys Glu Asp Asp Gly Tyr Pro Ala Met
   385                 390                 395                 400
   Asn Leu Asp Ala Gly Arg Glu Ser Gly Pro Asn Ser Glu Asp Leu Leu
                   405                 410                 415
   Leu Val Thr Asp Pro Ala Phe Leu Ser Cys Gly Cys Val Ser Gly Ser
               420                 425                 430
   Gly Leu Arg Leu Gly Gly Ser Pro Gly Ser Leu Leu Asp Arg Leu Arg
           435                 440                 445
   Leu Ser Phe Ala Lys Glu Gly Asp Trp Thr Ala Asp Pro Thr Trp Arg
       450                 455                 460
   Thr Gly Ser Pro Gly Gly Gly Ser Glu Ser Glu Ala Gly Ser Pro Pro
   465                 470                 475                 480
   Gly Leu Asp Met Asp Thr Phe Asp Ser Gly Phe Ala Gly Ser Asp Cys
                   485                 490                 495
   Gly Ser Pro Val Glu Thr Asp Glu Gly Pro Pro Arg Ser Tyr Leu Arg
               500                 505                 510
   Gln Trp Val Val Arg Thr Pro Pro Pro Val Asp Ser Gly Ala Gln Ser
           515                 520                 525
   Ser
<210> 11
   <211> 20
   <212> DNA
   <213> Homo sapiens
<400> 11
   agcatcaagc cggctccccc 20
<210> 12
   <211> 20
   <212> DNA
   <213> Homo sapiens
<400> 12
   ctccattcac tccaggtccc 20
<210> 13
   <211> 20
   <212> DNA
   <213> Mus musculus
<400> 13
   ttgaacgtga ctgtggcctt 20
<210> 14
   <211> 20
   <212> DNA
   <213> Mus musculus
<400> 14
   tgaatgaagt gcctggctga 20
<210> 15
   <211> 40
   <212> DNA
   <213> Homo sapiens
<400> 15
   cacaaagctt cagtatgagc tgcagtacag gaaccgggga 40
<210> 16
   <211> 40
   <212> DNA
   <213> Homo sapiens
<400> 16
   cacaggatcc ctttaactcc tctgactggg tctgaaagat 40